(19) Europäisches Patentamt
European Patent Office
Office européen des brevets

(11) **EP 2 364 106 B1**

(12) **EUROPÄISCHE PATENTSCHRIFT**

(45) Veröffentlichungstag und Bekanntmachung des Hinweises auf die Patenterteilung:
**26.02.2014 Patentblatt 2014/09**

(21) Anmeldenummer: **09782578.0**

(22) Anmeldetag: **03.09.2009**

(51) Int Cl.:
***A61B 5/00*** (2006.01)

(86) Internationale Anmeldenummer:
**PCT/EP2009/061419**

(87) Internationale Veröffentlichungsnummer:
**WO 2010/026197 (11.03.2010 Gazette 2010/10)**

(54) **WELLENLÄNGENABSTIMMBARE LICHTQUELLE**

WAVELENGTH-TUNABLE LIGHT SOURCE

SOURCE LUMINEUSE RÉGLABLE EN LONGUEUR D'ONDE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **03.09.2008 DE 102008045634**

(43) Veröffentlichungstag der Anmeldung:
**14.09.2011 Patentblatt 2011/37**

(73) Patentinhaber: **Lightlab Imaging, Inc.**
**Westford, MA 01886 (US)**

(72) Erfinder:
• **HUBER, Robert Alexander**
**83530 Schnaitsee (DE)**
• **EIGENWILLIG, Christoph**
**86807 Buchloe (DE)**
• **BIEDERMANN, Benjamin**
**81369 München (DE)**

(74) Vertreter: **Kopf, Korbinian Paul**
**Maiwald Patentanwalts GmbH**
**Elisenhof**
**Elisenstrasse 3**
**80335 München (DE)**

(56) Entgegenhaltungen:
**US-A1- 2002 054 614     US-A1- 2003 179 790**
**US-A1- 2005 078 716     US-A1- 2005 265 402**
**US-A1- 2008 165 366**

• **EIGENWILLIG C M ET AL: "Wavelength swept ASE source" PROCEEDINGS OF THE SPIE - THE INTERNATIONAL SOCIETY FOR OPTICAL ENGINEERING SPIE - THE INTERNATIONAL SOCIETY FOR OPTICAL ENGINEERING USA LNKD- DOI:10.1117/12.831831, Bd. 7372, 2009, XP002586268 ISSN: 0277-786X**

**Beschreibung**

Es wird eine wellenlängenabstimmbare Lichtquelle angegeben.

Spektral schmalbandige, wellenlängenabstimmbare Lichtquellen finden beispielsweise in der Medizin, insbesondere bei der optischen Kohärenztomographie, Anwendung. Über die optische Kohärenztomographie können zwei- oder dreidimensionale Abbildungen, beispielsweise von menschlichem Gewebe, erstellt werden. Ein wichtiger Aspekt solcher Abbildungen ist die erzielbare Auflösung, unter anderem also die Anzahl an Bildpunkten, die eine Abbildung ergeben. Hierdurch wird die Qualität einer Abbildung und beispielsweise deren diagnostische Verwendbarkeit maßgeblich beeinflusst. Neben einer hohen Detailwiedergabe ist auch die Zeitdauer, die für das Erstellen einer Abbildung benötigt wird, von Bedeutung. Um etwa die Belastung eines Patienten zu verringern, ist die für das Erstellen einer Abbildung benötigte Zeitdauer möglichst zu reduzieren. Die kombinierte Anforderung aus hoher Auflösung und geringer Zeitdauer bedeutet, dass eine hohe Datenrate ermöglicht sein soll. Dies stellt insbesondere hohe Anforderungen an eine für ein tomographisches Verfahren verwendbare Lichtquelle.

Eine Möglichkeit, eine schmalbandige, abstimmbare Lichtquelle zu realisieren, besteht darin, eine spektral breitbandige Strahlung, die etwa von einer Glühlampe oder Bogenlampe emittiert wird, nachfolgend zu filtern. Durch diese Methode sind allerdings keine hohen Strahlungsleistungen nach dem Durchlaufen des Filters erreichbar. Hat die Lichtquelle beispielsweise eine Bandbreite von 100 nm und der Filter eine Bandbreite von 0,1 nm, so beträgt der Verlust durch das Filtern zirka 99,9 %, entsprechend einer Abschwächung um einen Faktor 1000.

Eine weitere Möglichkeit, eine abstimmbare Lichtquelle zu realisieren, bildet ein abstimmbarer Laser. Der Laser weist hierbei ein Lasermedium, einen Resonator und einen abstimmbaren optischen Filter auf. Das Lasermedium kann spektral breitbandig verstärken. Der abstimmbare Filter ist im Resonator angeordnet. Es wird also vom Verstärkermedium nur Licht verstärkt, das den optischen Filter passiert und zum Verstärkermedium gelangt. Da ein Laser auf Verstärkung der spontanen Emission beruht, ist die vom Verstärkermedium emittierte Strahlung rückgekoppelt bezüglich der zum Verstärkermedium gelangenden Strahlung. Die Abstimmgeschwindigkeit des Filters ist, neben anderen Faktoren, insbesondere von der Länge des Resonators abhängig. Je größer die Länge des Resonators, desto geringer die erzielbare Abstimmgeschwindigkeit. Eine Verringerung der Resonatorlänge kann allerdings erhöhtes Intensitätsrauschen des Lasers verursachen und in einem größeren Frequenzabstand der Moden des Lasers resultieren. Hierdurch kann der maximale Messbereich in Anwendungen der optischen Kohärenztomographie, kurz OCT, begrenzt sein.

Eine andere Möglichkeit, eine abstimmbare Lichtquelle zu realisieren, besteht in einem Fourierdomänen-modengekoppelten Laser, englisch Fourier Domain Mode-Locked Laser, kurz FDML-Laser. Ein solcher Laser weist in einem Resonator mit einer großen Länge ein Verstärkermedium und mindestens einen abstimmbaren Filter auf. Die Abstimmgeschwindigkeit des Filters ist hierbei auf die Länge des Resonators angepasst. Mit anderen Worten ist der Filter für eine bestimmte Wellenlänge nach einer Zeit wieder transmittierend, die das Licht dieser Wellenlänge benötigt, um den Resonator einmal zu durchlaufen. Aufgrund dieses Funktionsprinzips und der hohen Lichtgeschwindigkeit weisen typische FDML-Laser Resonatorlängen im Bereich mehrerer 100 m bis hin zu einigen km auf. Hieraus ergeben sich Begrenzungen eines solchen FDML-Lasers bezüglich Kompaktheit und zugänglichem Wellenlängenbereich.

In der Druckschrift US 2008/0165366 A1 ist eine Methode und ein Apparat zur optischen Kohärenztomographie mit durchstimmbaren Lichtquellen angegeben. Die US 2008/0165366 A1 beschreibt eine Vorrichtung mit einem einstellbaren Laser, bei dem die Wellenlänge abstimmbar ist. Außerdem ist ein einstellbares Filterelement vorgesehen, das optisch mit der Ausgangsquelle verbunden ist. Außerdem ist ein Verstärkungselement, bzw. Verstärkermedium vorgesehen, das nach dem Filterelement angeordnet ist und die erzeugte Strahlung der Ausgangsquelle teilweise verstärkt.

Eine zu lösende Aufgabe besteht darin, eine wellenlängenabstimmbare Lichtquelle mit einer hohen Abstimmgeschwindigkeit anzugeben.

Diese Aufgabe wird durch eine Lichtquelle nach Anspruch 1 erreicht. Beispielhafte Ausführungsformen sind in den abhängigen Ansprüchen dargestellt.

Gemäß zumindest einer Ausführungsform der Lichtquelle weist diese ein wellenlängenselektives erstes Filterelement auf. Das erste Filterelement ist insbesondere der Ausgangsquelle optisch nachgeordnet. "Nachgeordnet" bedeutet, dass von der Ausgangsquelle emittierte Strahlung zum Filterelement gelangt. Das Filterelement wirkt filternd bezüglich der von der Ausgangsquelle emittierten Strahlung. Das heißt, der überwiegende Teil der von der Ausgangsquelle emittierten Strahlung außerhalb eines bestimmten Spektralbereichs kann das erste Filterelement nicht passieren. "Überwiegend" bedeutet, dass mehr als 80 %, bevorzugt mehr als 96 % der Strahlung außerhalb dieses Spektralbereichs das erste Filterelement nicht passieren kann.

Gemäß zumindest einer Ausführungsform der Lichtquelle weist diese ein erstes Verstärkermedium auf. Das erste Verstärkermedium ist insbesondere dem ersten Filterelement optisch nachgeordnet. Es gelangt also Strahlung, die das erste Filterelement passiert, zum ersten Verstärkermedium. Das erste Verstärkermedium ist dazu geeignet, im Betrieb die Strahlung, die das erste Filterelement passiert und zum ersten Verstärkermedium gelangt, mindestens teilweise zu verstärken. Das erste Verstärkermedium ist zum Beispiel ein Halbleiterverstärker, englisch Semiconductor Optical Amplifier, kurz SOA.

Gemäß zumindest einer Ausführungsform der Lichtquelle weist diese mindestens ein wellenlängenselektives zweites Filterelement auf. Das zweite Filterelement ist insbesondere dem ersten Verstärkermedium optisch nachgeordnet. Zum zweiten Filterelement gelangt also Strahlung, die das erste Verstärkermedium durchlaufen hat. Bevorzugt gelangt zum zweiten Filterelement nur solche Strahlung, die vom ersten Verstärkermedium verstärkt wurde. Verstärkt bedeutet hierbei insbesondere, dass eine spektrale Leistungsdichte nach Durchlaufen des ersten Verstärkermediums höher ist als eine spektrale Leistungsdichte der Strahlung vor Durchlaufen des ersten Verstärkermediums.

Gemäß zumindest einer Ausführungsform des ersten und/oder mindestens einen zweiten Filterelements beruht die wellenlängenselektive, filternde Wirkung des zweiten Filterelements auf Absorption, Reflexion oder Diffraktion. Zum Beispiel transmittiert das Filterelement Strahlung in einem bestimmten Wellenlängenbereich, während eine Strahlung mit anderen Wellenlängen reflektiert, absorbiert und/oder gebeugt wird.

Gemäß zumindest einer Ausführungsform der Lichtquelle weisen erstes Filterelement und zweites Filterelement einen optischen Abstand L zueinander auf. Unter dem optischen Abstand L ist die Weglänge der Strahlung gemeint, die diese in der Lichtquelle zu durchlaufen hat, um vom ersten Filterelement zum zweiten Filterelement zu gelangen. Der optische Brechungsindex eines Mediums, das die Strahlung auf dem Weg vom ersten Filterelement zum zweiten Filterelement durchläuft, ist hierbei zu berücksichtigen. Der optische Abstand L ist also insbesondere das Integral über Lichtweg und Brechungsindex.

Gemäß zumindest einer Ausführungsform der Lichtquelle weist diese eine Steuereinheit auf. Über die Steuereinheit ist das erste und das mindestens eine zweite Filterelement abstimmbar. Das heißt, über die Steuereinheit kann die Wellenlänge beziehungsweise der Wellenlängenbereich eingestellt und abgestimmt werden, in dem Strahlung das erste und das mindestens eine zweite Filterelement passieren kann. Die Steuereinheit kann einen Frequenzgenerator und/oder einen Frequenzmodulator umfassen.

Gemäß zumindest einer Ausführungsform der Lichtquelle sind das erste und das mindestens eine zweite Filterelement für eine Teilstrahlung der von der Ausgangsquelle emittierten Strahlung passierbar. Es ist möglich, dass erstes und zweites Filterelement für Teilstrahlung im gleichen Spektralbereich, im Rahmen der Herstellungs- und Messgenauigkeit, passierbar sind. Mit anderen Worten umfasst die Teilstrahlung also mindestens einen bestimmten Teilwellenlängenbereich des von der Ausgangsquelle emittierten Wellenlängenbereichs. Die Teilstrahlung kann bevorzugt über mindestens 50 %, insbesondere über mindestens 75 % des von der Ausgangsquelle emittierten Wellenlängenbereichs verstärkt und/oder abgestimmt werden.

Gemäß zumindest einer Ausführungsform der Lichtquelle sind erstes und zweites Filterelement über die Steuereinheit derart abstimmbar, dass erstes und zweites Filterelement in einer zeitlichen Verzögerung T zueinander für die Teilstrahlung durchlässig sind. Die zeitliche Verzögerung T ist hierbei gleich dem Quotienten aus dem optischen Abstand L und der Vakuumlichtgeschwindigkeit c, wobei c = 299.792,458 km/s ist. Es gilt also:

$$T = L/c.$$

Mit anderen Worten stimmt die Steuereinheit die Filterelemente derart ab, dass Teilstrahlung einer bestimmten Wellenlänge beziehungsweise in einem bestimmten Wellenlängenbereich, die das erste Filterelement passiert und nach einer Laufzeit zum zweiten Filterelement gelangt, auch von diesem zweiten Filterelement transmittiert wird. Die zeitliche Verzögerung T entspricht also der Laufzeit des Lichts in der Lichtquelle vom ersten Filterelement zum zweiten Filterelement. Umfasst die Lichtquelle mehr als ein zweites Filterelement, so sind bevorzugt alle weiteren zweiten Filterelemente in entsprechender Weise von der Steuereinheit abstimmbar.

Gemäß zumindest einer Ausführungsform der Lichtquelle ist eine Toleranz oder eine Variation für das Einstellen der zeitlichen Verzögerung T derart gering, dass der vom ersten Filterelement transmittierte Wellenlängenbereich zu mindestens 0,1 %, bevorzugt zu mindestens 5 %, besonders bevorzugt zu mindestens 30 % mit dem vom mindestens einen zweiten Filterelement transmittierten Wellenlängenbereich übereinstimmt. Die zeitliche Verzögerung T berücksichtigt, stimmen also die vom ersten und vom mindestens einen zweiten Filterelement transmittierten Wellenlängenbereiche zu mindestens den genannten Werten überein.

Gemäß zumindest einer Ausführungsform der Lichtquelle ist die zeitlichen Verzögerung T gegenüber dem Quotienten aus L/c variiert, so dass die zeitliche Verzögerung T kleiner oder größer ist als der Quotient aus L/c. Diese Variation ist hierbei höchstens so groß, dass eine Zentralwellenlänge einer Filtertransmission des zweiten Filterelements von einer Zentralwellenlänge der Teilstrahlung höchstens um einen Faktor 10, bevorzugt höchstens um einen Faktor 1, besonders bevorzugt höchstens um einen Faktor 0,5 mal einer Filterbandbreite des zweiten Filterelements abweicht. Die Filterbandbreite ist beispielsweise die volle spektrale Breite auf halber Höhe einer maximalen Transmission, kurz FWHM. Ist zum Beispiel zu einem bestimmten Zeitpunkt die Zentralwellenlänge der am zweiten Filterelement anliegenden Teilstrahlung 1320 nm und die Filterbandbreite 1 nm, so liegt die Zentralwellenlänge der Filtertransmission des zweiten Filterelements zwischen einschließlich 1310 nm und 1330 nm, bevorzugt zwischen 1319 nm und 1321 nm, besonders bevorzugt zwischen 1319,5 nm und 1320,5 nm. Durch eine derartige Variation der zeitlichen Verzögerung T ist es zum Beispiel möglich, den Wellenlängenbereich der Teilstrahlung exakt einzustellen.

Zur Verbesserung von Filtereigenschaften eines Filterelements können zum Beispiel zwei direkt aufeinander folgende Filter zu einem Filterelement zusammengefasst werden. Dies ist etwa bei einer Vernier-Abstimmung der Fall.

In mindestens einer Ausführungsform der wellenlängenabstimmbaren Lichtquelle umfasst diese eine Ausgangsquelle, die im Betrieb zur Erzeugung einer elektromagnetischen Strahlung geeignet ist. Weiterhin weist die Lichtquelle ein wellenlängenselektives erstes Filterelement auf, das der Ausgangsquelle nachgeordnet ist. Außerdem beinhaltet die Lichtquelle ein erstes Verstärkermedium, das dem ersten Filterelement nachgeordnet ist und mindestens zur teilweisen Verstärkung der von der Ausgangsquelle emittierten Strahlung geeignet ist. Die Lichtquelle umfasst weiterhin mindestens ein wellenlängenselektives zweites Filterelement, das dem ersten Verstärkermedium nachgeordnet ist, wobei das zweite Filterelement zum ersten Filterelement einen optischen Abstand L aufweist. Über eine Steuereinheit, die die Lichtquelle aufweist, sind das erste und das mindestens eine zweite Filterelement abstimmbar. Die Filterelemente werden hierbei so abgestimmt, dass erstes und zweites Filterelement für eine Teilstrahlung der von der Ausgangsquelle emittierten Strahlung in einer zeitlichen Verzögerung T zueinander durchlässig sind, wobei die Verzögerung T gleich dem Quotienten aus dem optischen Abstand L und der Vakuumlichtgeschwindigkeit c ist.

Eine solche wellenlängenabstimmbare Lichtquelle ermöglicht es, hohe Abstimmgeschwindigkeiten bezüglich der Wellenlänge der von der Lichtquelle emittierten Teilstrahlung zu erzielen. Ebenso kann von der Lichtquelle spektral schmalbandige Teilstrahlung emittiert werden.

Einer solchen Lichtquelle liegt unter anderem folgende Erkenntnis zugrunde: Die Ausgangsquelle erzeugt im Betrieb der Lichtquelle elektromagnetische Strahlung, die einen gewissen Frequenzbereich überdeckt. Ein Teil der Strahlung in dem Frequenzbereich passiert das erste Filterelement und gelangt zum ersten Verstärkermedium. Der vom ersten Filterelement emittierte Spektralanteil wird im ersten Verstärkermedium verstärkt. Zur Unterdrückung von Strahlung, die andere Wellenlängen als die Teilstrahlung aufweist, durchläuft das vom Verstärkermedium emittierte Licht nachfolgend ein zweites Filterelement. Es sind also mindestens zwei Filterelemente kaskadiert oder in Serie angeordnet. Erstes und zweites Filterelement weisen einen optischen Abstand L zueinander auf. Durch diesen optischen Abstand L gelangt Licht der Teilstrahlung, das das erste Filterelement durchläuft, erst nach einer gewissen zeitlichen Verzögerung zum zweiten Filterelement. Werden erstes und zweites Filterelement synchron zueinander abgestimmt, das heißt, dass erstes und zweites Filterelement zeitgleich für einen gewissen Spektralbereich etwa transmittierend sind, so kann es vorkommen, dass die Teilstrahlung, die das erste Filterelement durchlaufen hat, das zweite Filterelement nicht mehr passieren kann, da dieses bereits auf einen anderen Spektralbereich abgestimmt ist. Bei einer derartigen Ansteuerung der Filterelemente ist somit die Abstimmgeschwindigkeit, mit der die Wellenlänge der Teilstrahlung abgestimmt werden kann, limitiert. Dadurch, dass erstes und zweites Filterelement zeitverzögert für einen bestimmten Spektralbereich passierbar sind, entfällt diese Limitation. Die zeitliche Verzögerung T, mit der die Filterelemente abgestimmt werden, entspricht dem Laufzeitunterschied, den die Teilstrahlung auf dem Weg vom ersten Filterelement zum zweiten Filterelement erfährt.

Gemäß zumindest einer Ausführungsform der Lichtquelle weist diese keinen Resonator mit einem Verstärkermedium auf. Es befindet sich also ein Verstärkermedium, das gepumpt wird, innerhalb des Resonators, wie zum Beispiel bei einem Laser. Es sind in diesem Sinne Bauteile, die zu einer Frequenzfilterung dienen und auf dem Fabry-Perot-Prinzip basieren, keine Resonatoren. Beispielsweise bei einem Laser ist der Resonator ein für die Funktionsweise des Lasers entscheidendes Element.

Gemäß zumindest einer Ausführungsform der Lichtquelle umfasst diese mindestens ein zweites Verstärkermedium. Bevorzugt basiert das zweite Verstärkermedium auf einem Halbleitermaterial. Insbesondere ist das zweite Verstärkermedium ein SOA. Durch die Verwendung eines zweiten Verstärkermediums kann die von der Lichtquelle emittierte Strahlungsleistung erhöht werden.

Gemäß zumindest einer Ausführungsform der Lichtquelle ist die Ausgangsquelle als Verstärkermedium, insbesondere als erstes Verstärkermedium verwendbar. Das bedeutet, dass von der Ausgangsquelle emittiertes Licht zum ersten Filterelement gelangt, das Filterelement passiert und anschließend die Ausgangsquelle erneut durchläuft. Beim erneuten Durchlaufen der Ausgangsquelle wird das zuvor gefilterte Licht verstärkt. Auch bei einem solchen Aufbau der Lichtquelle ist das erste Verstärkermedium dem ersten Filterelement bezüglich des Laufwegs des Lichts nachgeordnet. Durch die Verwendung der Ausgangsquelle auch als Verstärkermedium kann eine besonders kompakte Lichtquelle aufgebaut werden.

Gemäß zumindest einer Ausführungsform der Lichtquelle kann wenigstens ein Filterelement und/oder wenigstens ein Verstärkermedium mindestens zweifach von der Teilstrahlung durchlaufen werden. Mit anderen Worten wird die Teilstrahlung, die zum Beispiel das erste Filterelement durchlaufen hat und einen Teilwellenlängenbereich der von der Ausgangsquelle emittierten Strahlung umfasst, in der Lichtquelle so geführt, dass diese Teilstrahlung ein weiteres Mal etwa zum ersten Filterelement gelangt oder mindestens zweimal das erste und/oder zweite Verstärkermedium durchläuft. Durch eine solche Strahlführung in der Lichtquelle lässt sich die Anzahl an Komponenten der Lichtquelle verringern. Hierdurch ist ein kompakter und kostengünstiger Aufbau der Lichtquelle ermöglicht.

Gemäß zumindest einer Ausführungsform der Lichtquelle ist mindestens ein Filterelement und/oder mindestens ein Verstärkermedium mindestens vierfach von der Teilstrahlung durchlaufbar. Durch eine solche Führung der Teilstrahlung innerhalb der Lichtquelle lassen sich besonders viele Komponenten einsparen und die Kompaktheit der Lichtquelle kann erhöht werden.

Gemäß zumindest einer Ausführungsform der Lichtquelle umfasst diese mindestens zwei polarisationsselektive Elemente. Zumindest ein polarisationsselektives Element ist beispielsweise ein Polarisator, ein Brewster-Fenster oder ein polarisationsabhängig reflektierender Strahlteiler. Durch die Verwendung eines solchen Elements ist es effizient möglich, Komponenten der Lichtquelle mehrfach von der Teilstrahlung durchlaufen zu lassen.

Gemäß zumindest einer Ausführungsform der Lichtquelle umfasst diese mindestens ein erstes und/oder zweites Verstärkermedium, das polarisationsabhängig ausgestaltet ist. Das bedeutet, nur Teilstrahlung einer bestimmten Polarisationsrichtung wird vom Verstärkermedium effizient verstärkt. Durch ein solches Verstärkermedium ist es möglich, dass die Polarisationseigenschaften des von der Lichtquelle emittierten Lichts gezielt einstellbar sind.

Gemäß zumindest einer Ausführungsform der Lichtquelle weist diese mindestens zwei polarisationsselektive Elemente auf, wobei das erste und/oder das zweite Verstärkermedium polarisationsunabhängig verstärkend ist. Hierdurch ist es ermöglicht, dass über die polarisationsselektiven Elemente Licht unterschiedlicher Polarisationsrichtungen durch ein Verstärkermedium gelenkt wird und trotzdem im Verstärkermedium eine Verstärkung erfährt. Dies vereinfacht den Aufbau der Lichtquelle.

Gemäß zumindest einer Ausführungsform der Lichtquelle ist diese wellenlängenagil. Wellenlängenagil, oder äquivalent hierzu frequenzagil, bedeutet, dass die Lichtquelle eine spektral schmalbandige Strahlung zur Verfügung stellt und dass schnell und kontrollierbar verschiedene Wellenlängen beziehungsweise Wellenlängenbereiche der Teilstrahlung einstellbar sind. Hierdurch lassen sich, limitiert lediglich durch die spektrale Breite der von der Ausgangsquelle erzeugten Strahlung und durch die maximale Abstimmgeschwindigkeit der Filterelemente, beliebige Wellenformen und zeitliche Verläufe der Wellenlänge der Teilstrahlung erzeugen. Dies unterscheidet die Lichtquelle beispielsweise von einem Laser mit einem Resonator oder von einem FDML-Laser, bei dem der zeitliche Wellenlängenverlauf der emittierten Strahlung weitgehend durch den Aufbau des Lasers vorgegeben ist.

Gemäß zumindest einer Ausführungsform der Lichtquelle ist diese nicht-periodisch abstimmbar. Das heißt, der zeitliche Verlauf der Wellenlänge der Teilstrahlung, für den die Filterelemente passierbar sind, weist keine Periodizität auf. Das bedeutet, dass innerhalb einer gewissen Zeit, die wesentlich größer ist als eine Umlauf- oder Durchlaufzeit des Lichts durch die Lichtquelle, der zeitliche Verlauf der Wellenlänge der Teilstrahlung sich nicht beziehungsweise nicht periodisch wiederholt. Bevorzugt ist die Lichtquelle nicht-periodisch innerhalb eines Zeitbereichs von mindestens 100 $\mu$s, insbesondere von mindestens 10 ms oder von mindestens 1 s. Dadurch, dass die Lichtquelle nicht-periodisch abstimmbar ist, erhöhen sich die Einsatzmöglichkeiten der Lichtquelle.

Gemäß zumindest einer Ausführungsform der Lichtquelle sind die Ausgangsquelle, das mindestens eine Verstärkermedium und die Filterelemente wenigstens teilweise, bevorzugt vollständig, über Lichtleiter optisch miteinander verbunden. Die Lichtleiter können zum Beispiel als Glasfasern ausgestaltet sein. Durch die Verwendung von Lichtleitern ist die Stabilität der Lichtquelle, insbesondere gegenüber Umwelteinflüssen, erhöht.

Gemäß zumindest einer Ausführungsform der Lichtquelle ist das erste Filterelement und/oder das mindestens eine zweite Filterelement mit einem Faser-Fabry-Perot-Filter gestaltet. Ein solcher Filter umfasst zum Beispiel die Enden zweier einander gegenüberliegender Lichtleiter, wobei die Enden ein Fabry-Perot-Element ausbilden. Die Enden können verspiegelt sein. Über einen Piezo-Aktuator kann der Abstand zwischen den Enden, und somit der zu transmittierende Wellenlängenbereich, eingestellt werden. Derartige Filter sind kompakt aufgebaut und lassen sich mit hoher Abstimmgeschwindigkeit abstimmen. Bevorzugt sind alle Filterelemente als Faser-Fabry-Perot-Filter gestaltet.

Gemäß zumindest einer Ausführungsform der Lichtquelle ist das erste Filterelement und/oder das mindestens eine zweite Filterelement ein transmissiv-absorptiver Filter. Das heißt, die Teilstrahlung wird transmittiert und die restliche Strahlung wird im Wesentlichen absorbiert. Die Absorption der nicht-transmittierten Teilstrahlung beträgt bevorzugt mehr als 80 %, insbesondere mehr als 90 %. Durch die Verwendung eines solchen Filters ist eine Strahlung mit anderen Wellenlängen als die der Teilstrahlung effizient unterdrückbar.

Gemäß zumindest einer Ausführungsform der Lichtquelle umfasst das erste Filterelement und/oder das mindestens eine zweite Filterelement mindestens ein optisches Gitter oder ein optisches Prisma und/oder einen Polygonspiegel. Die Verwendung solcher Komponenten für mindestens ein Filterelement erhöht die Ausgestaltungsmöglichkeiten der Lichtquelle und erlaubt vielfältige Einsatzmöglichkeiten der Lichtquelle.

Gemäß zumindest einer Ausführungsform der Lichtquelle ist zumindest ein erstes und/oder zweites Verstärkermedium als Filterelement verwendbar. Das heißt zum Beispiel, der Spektralbereich, in dem das Verstärkermedium eine Verstärkung von Strahlung zeigt, ist spektral schmal. Mit anderen Worten wird vom Verstärkermedium nur Strahlung in einem engen Spektralbereich verstärkt. Ebenso ist es möglich, dass über ein Pumpen des Verstärkermediums in der Zeitdomäne eine filternde Wirkung bezüglich eines Spektralbereichs erzeugt wird, insbesondere falls die am Verstärkermedium anliegende, zu verstärkende Teilstrahlung bezüglich deren Wellenlänge einen zeitlichen Verlauf aufzeigt. Das Verstärkermedium wird in diesem Falle etwa nur in bestimmten Zeitbereichen beispielsweise elektrisch gepumpt.

Nur während dieses Zeitbereichs, in dem das Verstärkermedium gepumpt wird, kann Strahlung verstärkt werden. Ist das Verstärkermedium mit einem Halbleiterbauteil gebildet, so kann der Spektralbereich, in dem das Verstärkermedium eine Verstärkung zeigt, alternativ oder zusätzlich durch eine Stärke des Stroms, mit dem das Verstärkermedium versorgt wird, eingestellt werden. Durch eine solche Ausgestaltung eines Verstärkermediums ist eine kompakte Lichtquelle realisierbar.

Gemäß zumindest einer Ausführungsform der Lichtquelle beträgt die spektrale Breite der von der Ausgangsquelle emittierten Strahlung mindestens 20 nm. Mit anderen Worten zeigt die Ausgangsquelle beispielsweise eine spontane Emission in einem Spektralbereich von mindestens 20 nm. Bevorzugt beträgt die spektrale Breite der Strahlung mindestens 70 nm, insbesondere mindestens 100 nm. Eine derartige spektral breitbandige Ausgangsquelle erhöht die Einsatzmöglichkeiten der Lichtquelle und ermöglicht eine hohe Auflösung im Bereich der optischen Kohärenztomographie.

Gemäß zumindest einer Ausführungsform der Lichtquelle liegt eine spektrale Breite der Teilstrahlung im Wertebereich zwischen 0,003 nm und 5 nm, insbesondere zwischen 0,05 nm und 1 nm. Es weisen also etwa die Filterelemente einen Transmissionsbereich auf, der der spektralen Breite der Teilstrahlung entspricht. Eine geringe spektrale Breite der Teilstrahlung ermöglicht es, mit der Lichtquelle eine hohe spektrale Auflösung zu erzielen.

Gemäß zumindest einer Ausführungsform der Lichtquelle beträgt eine optische Kohärenzlänge der Teilstrahlung mindestens 3 mm. Bevorzugt beträgt die Kohärenzlänge mindestens 5 mm. Dies bedeutet, dass die Teilstrahlung Wellenzüge ausbildet, deren Länge mindestens der Kohärenzlänge entspricht und dass innerhalb dieser Länge die Teilstrahlung zur Interferenz fähig ist. Die Kohärenzlänge bestimmt unter anderem eine maximale Tiefe, bis zu der im Rahmen einer optischen Tomographie Aufnahmen beispielsweise in einem Gewebe erstellt werden können. Über eine solche Kohärenzlänge können, im Rahmen einer tomographischen Anwendung, auch ausgedehntere Strukturen untersucht werden.

Gemäß zumindest einer Ausführungsform der Lichtquelle ist eine Zentralwellenlänge der Teilstrahlung während der zeitlichen Verzögerung T um mindestens ein Zehntel der spektralen Breite der Teilstrahlung abstimmbar. Bevorzugt ist die Zentralwellenlänge um mindestens die spektrale Breite der Teilstrahlung abstimmbar. Durch eine solche Lichtquelle sind hohe Datenraten etwa im Rahmen einer tomographischen Anwendung erzielbar.

Gemäß zumindest einer Ausführungsform der Lichtquelle beträgt eine Abstimmgeschwindigkeit mindestens eines der Filterelemente zumindest zeitweise wenigstens 0,5 nm/$\mu$s. Bevorzugt beträgt die Abstimmgeschwindigkeit mindestens 3,0 nm/$\mu$s. Eine solche Abstimmgeschwindigkeit ist bevorzugt für alle Filterelemente erzielbar. Über eine Lichtquelle mit derartig hohen Abstimmgeschwindigkeiten sind, beispielsweise im Rahmen einer tomographischen Anwendung, hohe Datenraten realisierbar.

Gemäß zumindest einer Ausführungsform der Lichtquelle beträgt eine Abstimmfrequenz des mindestens einen Filterelements, mit der die Wellenlänge der Teilstrahlung abgestimmt wird, zumindest zeitweise mindestens 40 kHz. Bevorzugt beträgt die Abstimmfrequenz mindestens 80 kHz, besonders bevorzugt mindestens 150 kHz. Das Filterelement kann mit einer solchen Frequenz insbesondere periodisch, zum Beispiel sinus- oder sägezahnförmig, abgestimmt werden. Eine solche Lichtquelle kann vielfältig in der Kohärenztomographie eingesetzt werden.

Gemäß zumindest einer Ausführungsform der Lichtquelle ist deren optische Länge kleiner oder gleich 300 m. Bevorzugt ist die optische Länge kleiner oder gleich 30 m. Unter optischer Länge ist diejenige Weglänge des Lichts zu verstehen, die das Licht von der Erzeugung in der Ausgangsquelle bis zum Verlassen der Lichtquelle zurücklegt. Der jeweilige Brechungsindex des Mediums, das das Licht durchläuft, ist hierbei zu berücksichtigen. Eine solche Lichtquelle ist kompakt aufgebaut und ermöglicht einen Einsatz in vielfältigen Spektralbereichen.

Gemäß zumindest einer Ausführungsform der Lichtquelle ist deren optische Länge größer als 10 cm, bevorzugt größer als 50 cm, insbesondere größer als 1 m.

Gemäß zumindest einer Ausführungsform der Lichtquelle enthält zumindest das erste und/oder das wenigstens eine zweite Verstärkermedium mindestens einen optischen Halbleiterverstärker. Bevorzugt sind alle Verstärkermedien, insbesondere alle Verstärkermedien und die Ausgangsquelle, mit einem Halbleiterverstärker gestaltet. Durch solche Verstärkermedien ist eine kompakte und zuverlässige Lichtquelle realisierbar.

Gemäß zumindest einer Ausführungsform der Lichtquelle enthält zumindest das erste und/oder das wenigstens eine zweite Verstärkermedium mindestens eine Seltene-Erden-dotierte Glasfaser oder sind durch eine solche gebildet. Es ist möglich, dass alle Verstärkermedien, insbesondere die Ausgangsquelle, eine solche Glasfaser umfassen.

Gemäß zumindest einer Ausführungsform der Lichtquelle ist zumindest ein erstes und/oder zweites Verstärkermedium abstimmbar bestrombar. Insbesondere ist das Verstärkermedium synchron zur spektralen Abstimmung der Filterelemente bestrombar. Durch eine abstimmbare Bestromung des Verstärkermediums kann eine gleichmäßige Ausgangsleistung bezüglich der Teilstrahlung der Lichtquelle gewährleistet werden.

Gemäß zumindest einer Ausführungsform der Lichtquelle ist die Ausgangsquelle ein Semiconductor Optical Amplifier und das erste und/oder das wenigstens eine zweite Verstärkermedium eine Seltene-Erden-dotierte Glasfaser. Eine Wellenlänge der von der Lichtquelle emittierten Strahlung liegt dann bevorzugt zwischen einschließlich 1000 nm und 1200 nm. Durch eine Verwendung von Strahlungsquellen verschiedenen Typs in der Lichtquelle können hohe optische Leistungen der Lichtquelle erzielt werden. Ebenso sind dann besonders kleine Schwankungen in der Leistung der von der Ausgangsquelle emittierten Strahlung erreichbar und ein Impulsbetrieb, der unerwünscht sein kann, ist unterdrückbar. Eine spektrale Formung der Strahlung kann dann ferner durch eine variable Bestromung der Ausgangsquelle ermöglicht sein.

Gemäß zumindest einer Ausführungsform der Lichtquelle gilt für eine Sättigungsleistung $P_{sat}$ des ersten und/oder des zweiten Verstärkermediums:

$$P_{sat} > 0,1 \; G \; P_A \; (B_P/B_A).$$

G ist hierbei ein Kleinverstärkungsfaktor des ersten beziehungsweise des zweiten Verstärkermediums, $B_P$ ist die spektrale Breite der Teilstrahlung und $B_A$ steht für die spektrale Breite der von der Ausgangsquelle emittierten Strahlung. $P_A$ ist eine Gesamtleistung der Strahlung der Ausgangsquelle. Obiger Zusammenhang ist bevorzugt dann erfüllt, wenn das erste Verstärkermedium und die Ausgangsquelle durch das gleiche Element gebildet sind. Durch eine solche Sättigungsleistung $P_{sat}$ des ersten Verstärkermediums ist es ermöglicht, dass die Teilstrahlung über einen weiten Spektralbereich der von der Ausgangsquelle emittierten Strahlung abstimmbar ist.

Gemäß zumindest einer Ausführungsform der Lichtquelle ist $P_{sat}$ mindestens einmal, insbesondere mindestens fünfmal so groß wie das Produkt aus Kleinverstärkungsfaktor G, spektraler Breite $B_P$ der Teilstrahlung geteilt durch spektrale Breite $B_A$ der Strahlung und der Gesamtleistung $P_A$ der Strahlung der Ausgangsquelle.

Gemäß zumindest einer Ausführungsform der Lichtquelle umfasst diese mindestens einen Detektor, bevorzugt einen wellenlängenselektiven Detektor. Der Detektor, wie etwa eine Fotodiode, ist beispielsweise über einen Farbfilter oder einen Interferenzfilter nur sensitiv in einem bestimmten Spektralbereich. Der Detektor ist bevorzugt nur für einen kleinen Spektralbereich der von der Ausgangsquelle emittierten Strahlung zugänglich. Durch einen solchen Detektor ist das von der Lichtquelle emittierte Licht charakterisierbar.

Gemäß zumindest einer Ausführungsform der Lichtquelle ist die Steuereinheit mit dem mindestens einen Detektor verbunden und ist die Verzögerung T durch ein Signal des Detektors einstellbar. Das heißt, die Steuereinheit empfängt und verarbeitet das wellenlängenselektive Signal des Detektors. Über die Steuereinheit und einen solchen Detektor ist die zeitliche Verzögerung T insbesondere automatisch einstellbar.

Gemäß zumindest einer Ausführungsform der Lichtquelle detektiert der Detektor einen Anteil der Teilstrahlung, die von einem zweiten Filterelement reflektiert wurde. Über einen solchen Detektor kann das Einstellen der zeitlichen Verzögerung T automatisiert werden und einfacher erfolgen.

Gemäß zumindest einer Ausführungsform der Lichtquelle umfasst diese mindestens einen Schließer, englisch Shutter. Über den Schließer kann die Lichtemission der Lichtquelle zeitweise verhindert werden. Während über den Schließer definierter Zeitintervalle gelangt somit kein Licht aus der Lichtquelle. Der Schließer kann ein oder mehrere Filterelemente umfassen. Bevorzugt ist der Schließer ebenfalls von der Steuereinheit angesteuert. Es ist möglich, dass eine Ansteuerung des Schließers synchron zu der Ansteuerung beispielsweise der Filterelemente erfolgt. Durch die Verwendung eines Schließers in der Lichtquelle erhöhen sich deren Einsatzmöglichkeiten.

Gemäß zumindest einer Ausführungsform der Lichtquelle ist diese dazu geeignet, Teilstrahlung im nahinfraroten Spektralbereich zu emittieren. Das heißt, die von der Lichtquelle emittierte Teilstrahlung umfasst Wellenlängen im Spektralbereich zwischen 780 nm und 3.000 nm, insbesondere zwischen 1.000 nm und 1.700 nm. Die Teilstrahlung kann auch ausschließlich nahinfrarotes Licht umfassen.

Gemäß zumindest einer Ausführungsform der Lichtquelle ist diese dazu geeignet, Teilstrahlung einer Wellenlänge von kleiner oder gleich 900 nm, bevorzugt von kleiner oder gleich 800 nm zu emittieren. Eine solche Lichtquelle ist dazu geeignet, beispielsweise in der Augendiagnostik eingesetzt zu werden. Zum Beispiel FDML-Laser weisen systembedingt Resonatorlängen von mehreren 100 m bis zu mehreren Kilometern auf. Daher können nur Glasfasern beziehungsweise Lichtleiter mit sehr geringer Abschwächung verwendet werden. In den Wellenlängenbereichen um 1.050 nm, 1.300 nm und 1.550 nm stellt dies keine signifikante Limitierung dar, da die Abschwächung verfügbarer Lichtleiter in diesem Bereich bei unter 1 dB/km liegt. In anderen Spektralbereichen, beispielsweise um 800 nm, ist die Abschwächung mit 3 dB/km signifikant größer. Neben der größeren Abschwächung ist auch die chromatische Dispersion im Spektralbereich beispielsweise um 800 nm deutlich größer als in den anderen vorher genannten Spektralbereichen. Durch die im Vergleich zu FDML-Lasern erheblich geringere Weglänge an zu durchlaufenden Material kann die Lichtquelle auch Strahlung bei und/oder unterhalb von 900 nm emittieren.

Gemäß zumindest einer Ausführungsform der Lichtquelle ist der optische Abstand zwischen der Ausgangsquelle und dem ersten Verstärkermedium möglichst groß gewählt. Dies gilt bevorzugt, falls die Ausgangsquelle als erstes Verstärkermedium eingesetzt wird und/oder eine Gesamtverstärkung der Strahlung der Ausgangsquelle mindestens 40 dB beträgt. Gesamtverstärkung bezieht sich hierbei auf einen zweifachen Durchgang der Strahlung durch die auch als erstes Verstärkermedium dienende Ausgangsquelle. Möglichst groß kann bedeuten, dass der optische Abstand zwischen der Ausgangsquelle und dem ersten Verstärkermedium mindestens dem 0,2-fachen Quotienten aus der Vakuumlichtgeschwindigkeit c und einer Filterwechselzeit entspricht, insbesondere mindestens dem 0,3-fachen, bevorzugt mindestens dem 0,5-fachen dieses Quotienten. Die Filterwechselzeit ist hierbei zum Beispiel eine Periodendauer der Wellenlängenabstimmung, multipliziert mit der Filterbandbreite des Filters und dividiert durch die spektrale Breite der von der Lichtquelle emittierten Strahlung.

Gemäß zumindest einer Ausführungsform der Lichtquelle umfasst diese mindestens eine Verzögerungsstrecke. Die Verzögerungsstrecke weist eine größere optische Länge auf als eine Überbrückungsstrecke, die der Verzögerungsstrecke parallel geschaltet ist. Zum Beispiel sind die Verzögerungsstrecke und die Überbrückungsstrecke über Faserkoppler oder über Strahlteiler verzweigt und/oder miteinander verbunden.

Gemäß zumindest einer Ausführungsform der Lichtquelle befindet sich die Verzögerungsstrecke, in Laufrichtung der Strahlung gesehen, nach dem letzten der Filterelemente und bevorzugt vor dem zweiten Verstärkermedium.

Gemäß zumindest einer Ausführungsform der Lichtquelle befindet sich die Verzögerungsstrecke, in Laufrichtung der Strahlung gesehen, zwischen zwei aufeinander folgenden Filterelementen.

Einige Anwendungsbereiche, in denen eine Lichtquelle gemäß einem oder mehrerer der beschriebenen Ausführungsbeispiele eingesetzt werden kann, sind die biomedizinische Bildgebung, etwa in den Bereichen Ophthalmologie, Kardiologie oder Gastroenterologie, die Profilometrie mit Subnanometerauflösung etwa im Maschinenbau, die Sensorik, insbesondere das Auslesen von Faser-Bragg-Gittern, die verteilte Temperaturmessung, die verteilte Spannungsmessung insbesondere im mechanischen Bereich, Vektor-Sonar, Verschlüsselung einer optischen Kommunikationsleitung oder Überwachung/Management von Telekommunikationsnetzwerken.

Nachfolgend wird eine hier beschriebene Lichtquelle anhand von Ausführungsbeispielen näher erläutert. Gleiche Bezugszeichen geben dabei gleiche Elemente in den einzelnen Figuren an. Es sind dabei jedoch keine maßstäblichen Bezüge dargestellt, vielmehr können einzelne Elemente zum besseren Verständnis übertrieben groß dargestellt sein.

Es zeigen:

Figur 1          eine schematische Darstellung eines Ausführungsbeispiels einer hier beschriebenen Lichtquelle,

Figur 2          eine schematische Darstellung eines abstimmbaren Lasers gemäß dem Stand der Technik,

Figur 3          eine schematische Darstellung eines Umlaufschemas eines abstimmbaren Lasers gemäß dem Stand der Technik,

Figur 4          eine schematische Darstellung eines Ausführungsbeispiels einer hier beschriebenen Lichtquelle, bei der Ausgangsquelle und erstes Verstärkermedium vom selben Element gebildet sind,

Figur 5          eine Darstellung eines zeitlich gemittelten Emissionsspektrum eines Ausführungsbeispiels einer hier beschriebenen Lichtquelle,

Figuren 6 bis 8          Darstellungen der Ausgangsleistung, der Treiber- und Interferenzsignale eines Ausführungsbeispiels einer hier beschriebenen Lichtquelle,

Figur 9          eine Darstellung der mittleren integrierten Linienbreite eines Ausführungsbeispiels einer hier beschriebenen Lichtquelle,

Figuren 10 bis 19          schematische Darstellungen von Ausführungsbeispielen von hier beschriebenen Lichtquellen mit mehrfachem Durchgang der Teilstrahlung durch einzelne Komponenten der Lichtquelle,

Figur 20          eine schematische Darstellung zur Synchronisation mehrerer Filterelemente eines Ausführungsbeispiels einer hier beschriebenen Lichtquelle,

Figur 21 und 22          schematische Darstellungen von Signalen von Detektoren eines Ausführungsbeispiels einer hier beschriebenen Lichtquelle,

Figur 23          eine schematische Darstellung einer Anordnung von Filterelementen eines hier beschriebenen Ausführungsbeispiels einer Lichtquelle, und

Figuren 24 und 25          schematische Darstellungen von Modulen für weitere Ausführungsbeispiele von hier beschriebenen Lichtquellen.

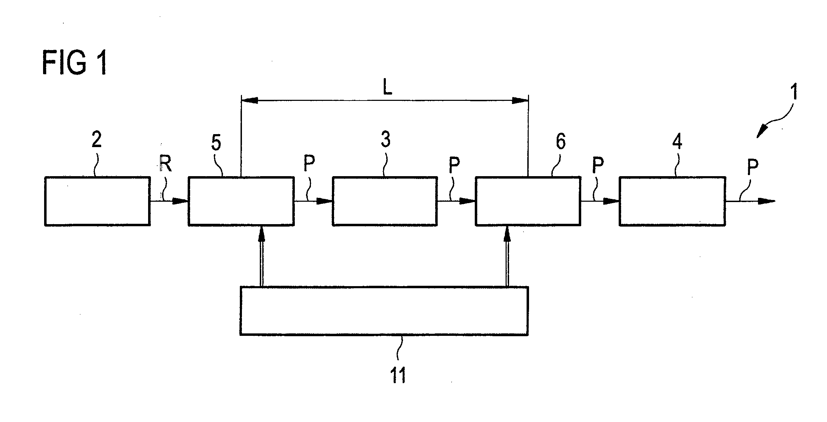

In Figur 1 ist ein Ausführungsbeispiel einer hier beschriebenen wellenlängenabstimmbaren Lichtquelle 1 dargestellt. Von einer Ausgangsquelle 2 wird eine spektral breitbandige Strahlung R emittiert. Die Strahlung R durchläuft nachfolgend ein erstes Filterelement 5, das von einer Teilstrahlung P, die einen Teil und einen spektralen Ausschnitt der Strahlung R darstellt, passiert wird. Die Teilstrahlung P wird in einem ersten Verstärkermedium 3 verstärkt. Das erste Verstärkermedium 3 ist dazu ausgestaltet, dass der überwiegende oder der gesamte Spektralbereich, den die Strahlung R der Ausgangsquelle abdeckt, auch im ersten Verstärkermedium 3 verstärkt werden kann. Dadurch ist die Teilstrahlung P über den überwiegenden oder gesamten Spektralbereich der Strahlung R abstimmbar.

Damit einher geht aber auch, dass mit der Verstärkung der Teilstrahlung P auch ein kleiner Anteil einer spektral breitbandigen Untergrundstrahlung erzeugt wird. Um diese störende Untergrundstrahlung zu unterdrücken, ist dem ersten Verstärkermedium 3 ein zweites Filterelement 6 nachgeordnet, das die verstärkte Teilstrahlung P passieren lässt.

Erstes Filterelement 5 und zweites Filterelement 6 weisen einen optischen Abstand L zueinander auf. Beide Filterelemente 5, 6 sind wellenlängenabstimmbar, die Abstimmung erfolgt über die Steuereinheit 11. Ein Treibersignal, symbolisiert durch zwei verschiedene Doppellinien, das am ersten Filterelement 5 anliegt, liegt am zweiten Filterelement 6 mit einer zeitlichen Verzögerung T an, die dem optischen Abstand L geteilt durch die Vakuumlichtgeschwindigkeit c entspricht. Der Laufzeitunterschied der Teilstrahlung P auf dem Weg von erstem Filterelement 5 zum zweiten Filterelement 2, der bei einem Laufweg von 1 m durch einen Lichtleiter zirka 5 ns beträgt, ist also durch die Steuereinheit 11 kompensiert. Hierdurch ist vermieden, dass bei hohen Abstimmgeschwindigkeiten der kaskadiert angeordneten Filterelemente 5, 6 die Teilstrahlung P durch aufeinander folgende Filterelementen 5, 6 eine deutliche Intensitätsminderung erfährt.

Optional ist dem zweiten Filterelement 6 ein zweites Verstärkermedium 4 nachgeordnet, das die Teilstrahlung P nochmals verstärkt. Hierdurch sind hohe Ausgangsleistungen der Teilstrahlung P erreichbar. Das zweite Verstärkermedium 4 weist im Wesentlichen denselben spektralen Verstärkungsbereich auf wie das erste Verstärkermedium 3. Ohne das zweite Filterelement 6 würde die Untergrundstrahlung vom zweiten Verstärkermedium 4 ebenfalls verstärkt werden, die Teilstrahlung P wäre dann nicht oder nur eingeschränkt spektral abstimmbar und/oder die Teilstrahlung P würde einen spektral breiten Untergrund aufweisen.

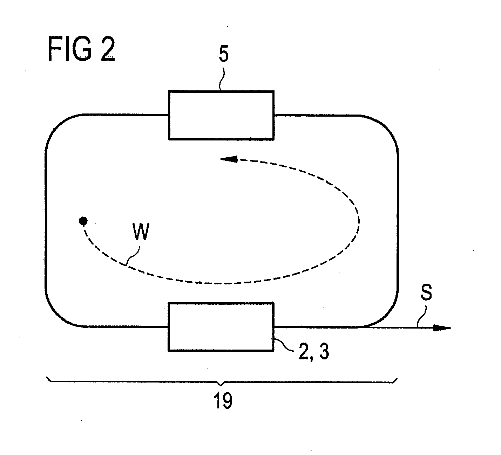

In Figur 2 ist ein abstimmbarer Laser gemäß dem Stand der Technik dargestellt. Der Laser weist ein Verstärkermedium 3 auf, das gleichzeitig als Ausgangsquelle 2 dient. Das Verstärkermedium 3 befindet sich, wie auch ein Filter 5, in einem schematisch als ringartige Struktur dargestellten Resonator 19. Von dem Laser erzeugte Strahlung S ist als Pfeil-Linie gezeichnet, ein Lichtweg W der Strahlung S im Resonator 19 ist als eine Strich-Pfeil-Linie illustriert. Der Filter 5 ist beispielsweise als ein Gitterspiegel oder als ein Faser-Fabry-Perot-Filter gestaltet.

Eine Umlaufzeit $T_U$ des Lichts im Resonator 19 entspricht einer Resonatorlänge $L_U$ geteilt durch die Vakuum-Lichtgeschwindigkeit c, wobei bei der Resonatorlänge $L_U$ die Brechungsindices der Medien, die das Licht durchläuft, zu berücksichtigen sind.

Im Resonator 19 des Lasers können nur solche Wellenlängen umlaufen, die vom Filter 5 nicht absorbiert werden. Der Laser emittiert im Normalbetrieb Licht mit einer Wellenlänge, die in etwa einem Verlustminimum beziehungsweise einem Maximum eines Transmissionsfenster $B_P$ des Filters 5 entspricht. Um einen kontinuierlich abstimmbaren Laser zu erhalten, wird die Wellenlänge des Transmissionsfensters $B_P$ des Filters 5 verändert. Diese Methode ist praktikabel, so lange das Licht mehrere Umläufe im Resonator 19 absolvieren kann, bevor das Transmissionsfenster $B_P$ des Filters 5 zu einer anderen Wellenlänge hin verschoben wird. Durch diese Bedingung ist eine Abstimmgeschwindigkeit des Filters 5 limitiert.

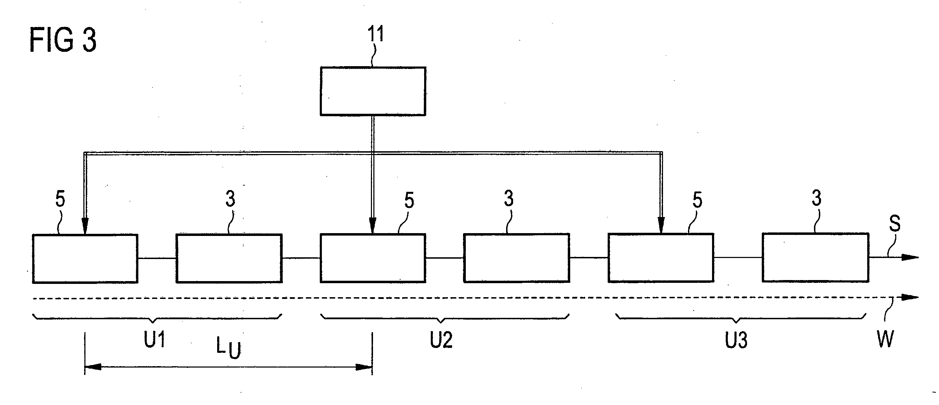

In Figur 3 ist das Prinzip eines abstimmbaren Lasers gemäß Figur 2 in einer weiteren Darstellung veranschaulicht. Das zyklische Durchlaufen des Resonators 19 von der Strahlung S ist als lineare Abfolge illustriert. Die Strahlung S durchläuft in einem ersten Umlauf U1, in Figur 2 symbolisiert durch eine geschweifte Klammer, das Filterelement 5 und anschließend das Verstärkermedium 3. Nachfolgend, in einem zweiten Umlauf U2 durchläuft die Strahlung S erneut das Filterelement 5 und wiederum das Verstärkermedium 3. Entsprechendes erfolgt in einem dritten Umlauf U3 und in eventuellen weiteren Umläufen.

Verstärkermedium 3 und Filterelement 5 werden also abwechselnd durchlaufen. Die Zeit, die die Strahlung S pro Umlauf benötigt, entspricht der optischen Länge $L_U$ des Resonators 19 und ist gleich der optischen Wegstrecke zwischen zwei aufeinander folgenden Durchgängen durch das Filterelement 5, geteilt durch die Vakuumlichtgeschwindigkeit c.

In Figur 3 ist, wie beschrieben, eine schematische Darstellung des Lichtwegs W gemäß Figur 2 angegeben, so dass dasselbe Filterelement 5 und dasselbe Verstärkermedium 3 zeitlich nacheinander immer wieder durchlaufen werden. Somit liegt an allen gezeichneten Filterelementen 5, da es sich immer um dasselbe Filterelement handelt, das gleiche Signal einer Steuereinheit 11 an, symbolisiert durch eine sich verzweigende Doppellinie. Alle Filterelemente 5 weisen also zu einem bestimmten Zeitpunkt einen gleichen, freien beziehungsweise passierbaren Wellenlängenbereich auf. Alternativ ist es ebenso möglich, dass eine Kette verschiedener Filterelemente 5 und verschiedener Verstärkerelemente 3 in alternierender Abfolge vorliegt, wobei zu einem bestimmten Zeitpunkt immer alle Filterelemente 5 für denselben Wellenlängenbereich passierbar sind.

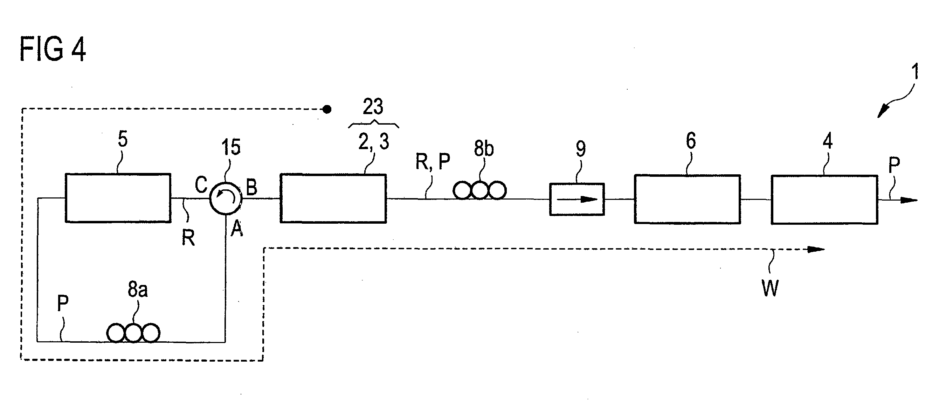

Beim Ausführungsbeispiel einer hier beschriebenen Lichtquelle gemäß Figur 4 weist die wellenlängenabstimmbare Lichtquelle 1 ein Element 23 auf, das gleichzeitig als Ausgangsquelle 2 und als erstes Verstärkermedium 3 dient. Im Betrieb der Ausgangsquelle 2 wird die Strahlung R erzeugt. Diese läuft, mindestens zum Teil, in Richtung zu einem optischen Zirkulator 15. Der Zirkulator 15 weist drei Anschlüsse A, B, C auf. Licht, das den Zirkulator 15 durch Anschluss A betritt, wird an Anschluss B ausgegeben. Licht, das in den Anschluss B gelangt, wird am Anschluss C ausgegeben. Und Licht, das den Zirkulator über Anschluss C betritt, wird nicht weitergeleitet. Somit wirkt der Zirkulator 15 bezüglich Licht am Anschluss C als optischer Isolator.

Die Strahlung R der Ausgangsquelle 2 betritt den Zirkulator an Anschluss B und wird zum Anschluss C weitergeleitet. Nachfolgend durchläuft die Strahlung R, deren Gesamtleistung $P_A$ zirka 1 mW beträgt, das erste Filterelement 5. Die Teilstrahlung P der Strahlung R wird vom ersten Filterelement 5 transmittiert. Die Leistung dieser transmittierten Teilstrahlung P beträgt in etwa 3 $\mu$W. Zur Einstellung der Polarisation der Teilstrahlung P durchläuft diese einen Polarisationscontroller 8a. Nachfolgend betritt die Teilstrahlung P den Zirkulator 15 an Anschluss A und wird zum Anschluss B weitergeleitet. Daraufhin durchläuft die Teilstrahlung P das Element 23 beziehungsweise das erste Verstärkermedium 3, das eine polarisationsabhängige Verstärkung aufweist. Die Polarisation der Teilstrahlung P wird über den Polarisationscontroller 8a so eingestellt, dass eine maximale Verstärkung der Teilstrahlung P im Verstärkermedium 3 erfolgt.

Optional kann die vom ersten Verstärkermedium 3 verstärkte Teilstrahlung P einen weiteren Polarisationscontroller 8b durchlaufen. Diesem zweiten Polarisationscontroller 8b ist, ebenfalls optional, ein optischer Isolator 9 nachgeordnet, eine Durchlassrichtung des Isolators 9 ist durch einen Pfeil gekennzeichnet. Auf den Isolator 9 folgt das zweite Filterelement 6. Die Filterelemente 5, 6 werden derart von der nicht dargestellten Steuereinheit abgestimmt, dass der Laufzeitunterschied der Teilstrahlung P vom ersten Filterelement 5 zum zweiten Filterelement 6 ausgeglichen ist, so dass eine hohe Abstimmgeschwindigkeit bezüglich der Wellenlänge der Teilstrahlung P erzielbar ist.

Das zweite Filterelement 6 unterdrückt die von der Ausgangsquelle 2 emittierte, breitbandige Emission, die die Strahlung R bildet und in Richtung hin zum zweiten Filterelement 6 läuft. Somit gelangt diese Strahlung R, bis auf Strahlung im Spektralbereich der Teilstrahlung P, nicht zum zweiten Verstärkermedium 4. Über das zweite Verstärkermedium 4 wird die Teilstrahlung P nochmals verstärkt und verlässt nachfolgend die Lichtquelle 1.

Die einzelnen Komponenten der Lichtquelle 1 sind durch Lichtleiter, die mit Glaserfasern gestaltet sind, optisch verbunden.

Das Element 23 ist zum Beispiel als fasergekoppelter optischer Halbleiterverstärker gestaltet. Eine Sättigungsleistung $P_{sat}$ dieses Elements 23, also die maximale Lichtleistung, die dieses emittieren kann, beträgt einige 10 mW. Eine Zentralwellenlänge der Strahlung R liegt bei zirka 1310 nm. Das Element 23 zeigt eine polarisationsabhängige Verstärkung auf, wobei die Polarisationsabhängigkeit zirka 16 dB beträgt. Ein Kleinsignalverstärkungsfaktor G des Elements 23 beträgt etwa 25 dB, entsprechend in etwa einem Faktor 300.

Erstes Filterelement 5 und zweites Filterelement 6 sind Faser-basierte, abstimmbare Faser-Fabry-Perot-Filter, auch als Fiber-Fabry-Perot-Tunable Filter, kurz FFP-TF, bezeichnet. Die Filterelemente 5, 6 weisen eine Bandbreite von zirka 0,3 nm auf. Diese Bandbreite entspricht auch der spektralen Breite der Teilstrahlung P. Ein freier Spektralbereich der Filterelement 5, 6 beträgt zirka 130 nm. Innerhalb des freien Spektralbereichs können die Filterelemente 5, 6 abgestimmt werden. Die Filterelemente 5, 6 transmittieren jeweils eine Strahlung, die Wellenlängen der Teilstrahlung P aufweist. Andere Wellenlängen werden von den Filterelementen 5, 6 zurück in Richtung zum Element 23 reflektiert und vom Zirkulator 15 beziehungsweise vom Isolator 9 absorbiert. Die Filterelemente 5, 6 zeigen eine Unterdrückung, außerhalb des Spektralbereichs der Teilstrahlung P, von zirka 30 bis 40 dB.

Mit einer spektralen Breite des Transmissionsfensters $B_P$ von 0,3 nm, einer spektralen Breite der vom Element 23 emittierten Strahlung R von zirka 100 nm, einem Kleinsignalverstärkungsfaktor G von 25 dB und der Gesamtleistung $P_A$ der Strahlung R von zirka 1 mW beträgt das Produkt aus G, $P_A$ und ($B_P/B_A$) zirka 0,9 mW. Dieser Wert ist deutlich kleiner als die Sättigungsleistung $P_{sat}$ des Elements 23 von etwa 50 mW. Mit anderen Worten liegt die Leistung der Teilstrahlung P nach Durchlaufen des Elements 23 deutlich unter der Sättigungsleistung $P_{sat}$. Da das Element 23 nicht in Sättigung betrieben wird, ist die spontane Emission des Elements 23 nicht unterdrückt und eine gleichzeitige Verwendung als Ausgangsquelle 2, die eine spektral breitbandige Strahlung R emittiert, und als erstes Verstärkermedium 3 ist ermöglicht.

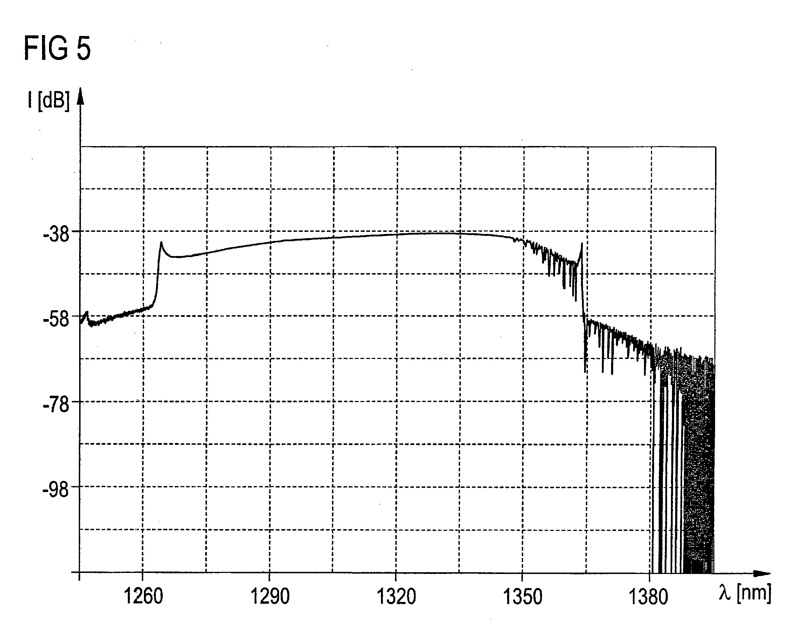

Figur 5 zeigt das zeitlich gemittelte Spektrum des von der in Figur 4 dargestellten Lichtquelle 1 emittierten Lichts. Das heißt, über die zu verschiedenen Zeiten emittierten Spektren der Teilstrahlung P ist zeitlich gemittelt, so dass der gesamte, von der abstimmbaren Lichtquelle 1 emittierte Spektralbereich dargestellt ist. Aufgetragen ist die Intensität I der Teilstrahlung P in dB gegenüber der Wellenlänge $\lambda$ in nm . Über einen Spektralbereich mit einer Breite von zirka 100 nm von etwa 1265 nm bis 1365 nm ist die spektrale Intensität I bis auf zirka 5 dB gleich bleibend. Die Lichtquelle 1 stellt also eine spektral breitbandige Lichtquelle 1 dar. Die Zentralwellenlänge liegt bei zirka 1310 nm. Die zeitlich gemittelte Leistung der von der Lichtquelle 1 emittierten Teilstrahlung P beträgt zirka 50 mW bis 100 mW.

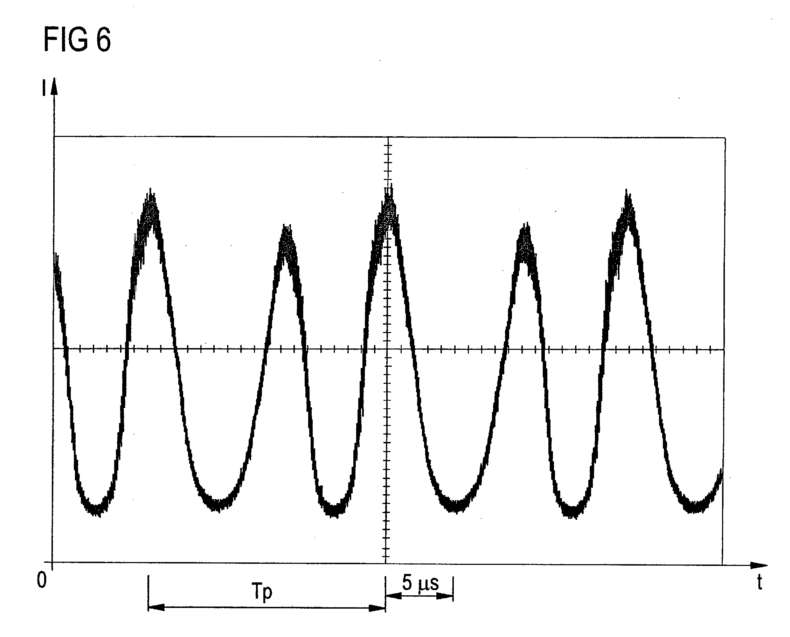

In Figur 6 ist die Intensität I der emittierten Teilstrahlung P linear gegenüber der Zeit t aufgetragen. Die Abstimmfrequenz der Filterelemente 5, 6 beträgt zirka 56 kHz, entsprechend einer Periodendauer $T_P$ von zirka 18 $\mu$s. Während einer Periodendauer wird, über das bidirektionale Verfahren des Faser-Fabry-Perot-Filters, während einer Periode jede Wellenlänge mehrmals eingestellt, ein- beziehungsweise mehrmals beim Vergrößern des Abstandes der den Faser-Fabry-Perot-Filter bildenden Enden der Lichtleiter, und einbeziehungsweise mehrmals beim Verringern des Abstandes. Hierdurch beträgt eine effektive Abstimmfrequenz der Teilstrahlung P zirka 112 kHz, entsprechend zweimal 56 kHz. Beim Vergrößern beziehungsweise Verkleinern des Abstandes der Filterelemente 5, 6 sind geringe Intensitätsschwankungen bemerkbar, das heißt, nur jedes zweite Maximum der in Figur 6 dargestellten Kurve zeigt gleiche Intensität.

Im Bereich der einzelnen Maxima der in Figur 6 dargestellten Kurve weist diese in etwa eine gaußförmige Gestalt auf. Diese Glockenkurven-artige Gestalt ist zum Beispiel für eine nachfolgende Fourier-Transformation der Intensität, etwa im Rahmen einer tomographischen Anwendung im Bereich der Medizin, von Vorteil. Um einen zeitlich gleichmäßigeren Verlauf der von der Lichtquelle 1 emittierten Leistung der Teilstrahlung P zu erzielen, kann optional ein Treiberstrom von erstem Verstärkermedium 3 und/oder zweitem Verstärkermedium 4 zeitabhängig eingestellt werden. Hierdurch ändert sich zeitabhängig auch die Verstärkung der Verstärkermedien 3, 4 und eine zeitabhängige Einstellung der Leistung der Teilstrahlung P ist möglich.

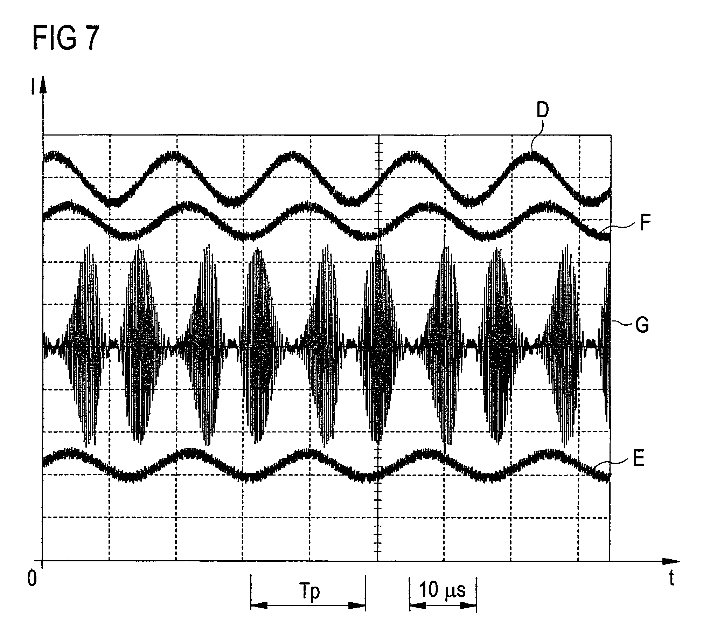

In Figur 7 sind die Intensitäten I der Treibersignale der Steuereinheit 11 für die Filterelemente 5, 6 sowie ein Interferogramm der Teilstrahlung P, jeweils linear aufgetragen gegenüber der Zeit t, dargestellt. Zum Beispiel ein Frequenzgenerator erzeugt ein harmonisches Signal mit einer Frequenz von 56 kHz, siehe die mit D gekennzeichnete Kurve. Dieses Signal wird verstärkt und mit einem Gleichstromsignal überlagert. Hieraus resultiert das Treibersignal, Kurve E, für das erste Filterelement 5. Ebenso wird aus dem Signal des Funktionsgenerators das Treibersignal für das zweite Filterelement 6, Kurve F, generiert. Die Treibersignale von erstem Filterelement 5 und zweitem Filterelement 6 weisen eine Phasenverschiebung zueinander auf, siehe Kurven E und F, die zirka einem Zwanzigstel der Periodendauer $T_P$ entspricht.

Weiterhin ist ein Interferogramm, Kurve G, dargestellt. Dieses Interferogramm ist am Ausgang eines Mach-Zehnder-Interferometers mit unterschiedlichen Armlängen, Differenz der Armlängen zirka 1 mm, mit einem differenziellen Detektor aufgenommen. Die Kurve G zeigt ein deutliches Oszillieren. Dieses Oszillieren der Kurve G resultiert aus dem zeitlichen Verlauf der Wellenlänge der von der Lichtquelle 1 emittierten Teilstrahlung P.

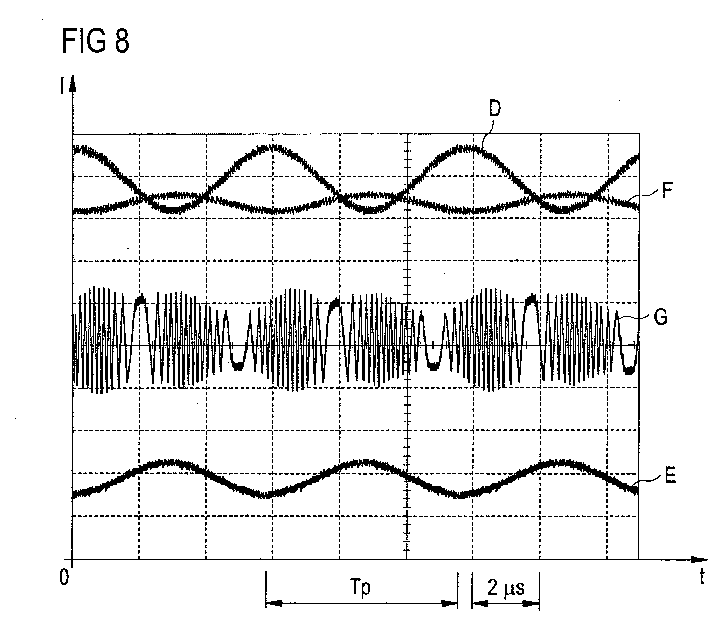

Figur 8 zeigt eine entsprechende Darstellung wie Figur 7. Allerdings beträgt die Treiberfrequenz 170 kHz, entsprechend 340000 Wellenlängendurchläufen pro Sekunde. Die Achse bezüglich der Zeit t ist entsprechend angepasst. Der Wellenlängenabstimmbereich, über den die Teilstrahlung P durchgestimmt ist, beträgt zirka 50 nm, im Vergleich zu etwa 100 nm gemäß Figur 7. Die Ausgangsleistung des von der Lichtquelle 1 emittierten Lichts, gemittelt über die Zeit, beträgt zirka 30 mW. Bedingt durch den kleineren Abstimmbereich von 50 nm und dem lineareren Verlauf der Intensitätskurve I in diesem engeren Wellenlängenbereich, vergleiche Figur 5, weist das Interferogramm, siehe Kurve G, eine gleichmäßigere Intensitätsverteilung auf, verglichen mit Figur 7.

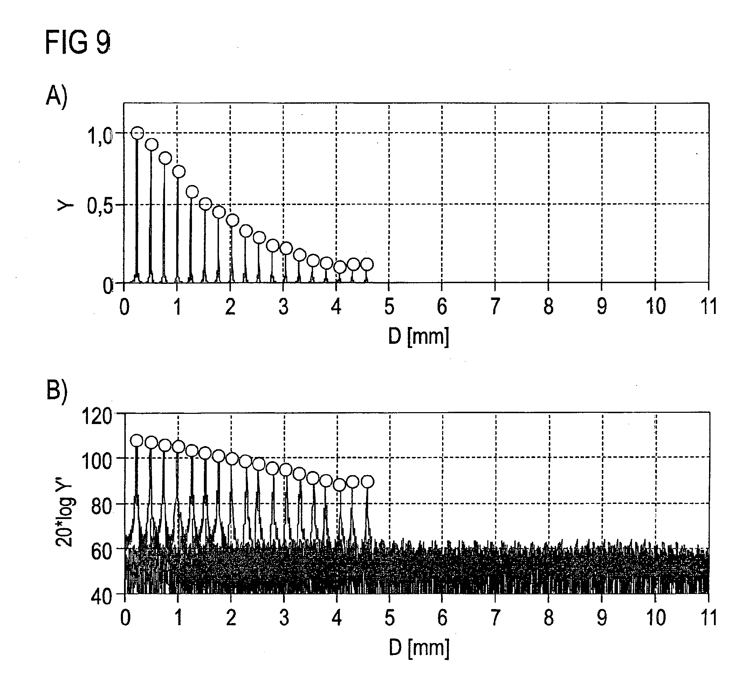

Figur 9 zeigt eine Punktverteilungsfunktion, englisch Point Spread Function, kurz PSF, in Analogie zu einer Anwendung für die optische Kohärenztomographie, gemessen an einem vereinfachten Aufbau. Aufgetragen ist, in beliebigen Einheiten, die Signalamplitude Y beziehungsweise Y', in Figur 9A linear und in Figur 9B logarithmisch, gegenüber einer Armlängendifferenz D eines Interferometers in mm. Bis zu einem Wert von zirka 3 mm beträgt die Abschwächung der Signale in etwa 20 dB. Dies bedeutet, bis zu einer optischen Schichtdicke von zirka 3 mm kann zum Beispiel im Rahmen einer optischen Kohärenztomographie-Anwendung ein Signal interferometrisch gewonnen werden. Die Armlängendifferenz ist auch ein Maß für die mittlere integrierte Linienbreite der Teilstrahlung P. Die aus der Messung ermittelte, mittlere integrierte Linienbreite stimmt mit der durch die Filterelemente 5, 6 vorgegebenen Linienbreite von 0,3 nm gut überein. Die Lichtquelle 1 ist also für beispielsweise tomographische Anwendungen geeignet.

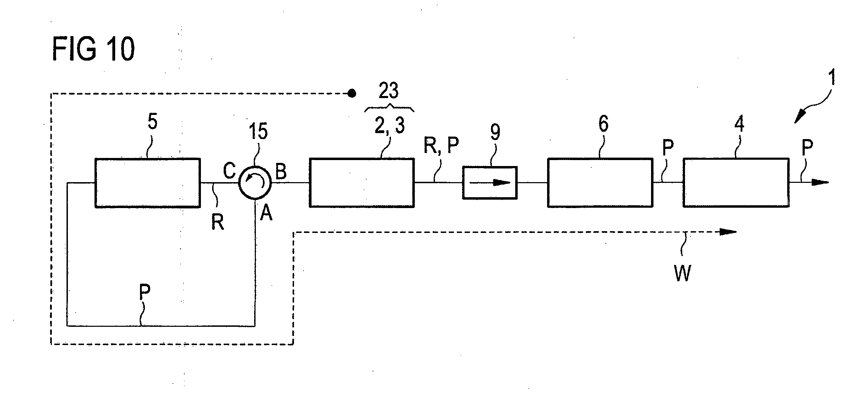

In den Figuren 10 bis 20 sind Ausführungsbeispiele von wellenlängenabstimmbaren Lichtquellen 1 gezeigt, bei denen mindestens eine Komponente mindestens zweifach von der Strahlung R beziehungsweise der Teilstrahlung P durchlaufen wird. Die Steuereinheit ist jeweils nicht dargestellt.

Das Ausführungsbeispiel der Lichtquelle 1 gemäß Figur 10 entspricht im Wesentlichen dem in Figur 4 gezeigten. Ausgangsquelle 2 und erstes Verstärkermedium 3 sind vom gleichen Element 23 gebildet, das eine polarisationsunabhängige Verstärkung aufzeigt. Hierdurch entfallen die Polarisationscontroller.

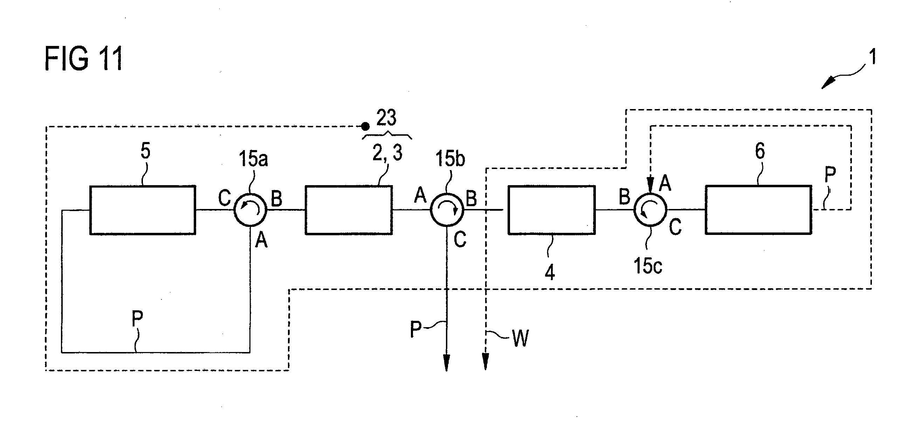

Beim Ausführungsbeispiel gemäß Figur 11 weist die Lichtquelle 1 drei Zirkulatoren 15a, 15b, 15c auf. Über die Zirkulatoren 15b, 15c ist ein zweimaliges Durchlaufen des ersten Verstärkermediums 3 sowie des zweiten Verstärkermediums 4 möglich. Der Lichtweg W ist durch eine Pfeil-Strich-Linie illustriert. Durch das zweimalige Durchlaufen des zweiten Verstärkermediums 4 ist eine hohe Leistung der Teilstrahlung P gewährleistet. Dies ermöglicht es beispielsweise, die spektrale Breite der Teilstrahlung P zu verringern.

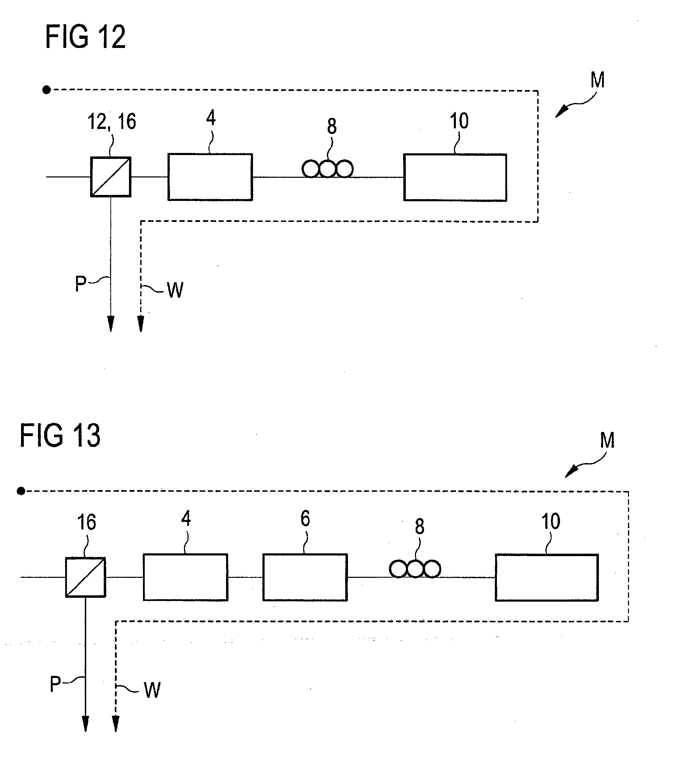

Figur 12 zeigt ein Modul M, das beispielsweise anstelle des zweiten Verstärkermediums 4 gemäß Figur 4 oder Figur 10 eingesetzt werden kann. Die bevorzugt linear polarisierte Teilstrahlung P wird durch ein polarisationsselektives Bauteil 12, das als Polarisationsstrahlteiler 16 gestaltet ist, transmittiert und durchläuft das zweite Verstärkermedium 4. Nachfolgend wird über den Polarisationscontroller 8 die Polarisation gedreht. Die Strahlung wird von einem Spiegel 10, der als metallischer Spiegel, dielektrischer Spiegel oder als Faraday-Spiegel gestaltet sein kann, reflektiert, durchläuft nochmals den Polarisationscontroller 8, wodurch die Polarisation effektiv zum Beispiel um 90° gedreht wird. Nachfolgend wird das zweite Verstärkermedium 4, das polarisationsunabhängig verstärkt, nochmals durchlaufen. Da, aufgrund des zweimaligen Durchlaufens der Polarisationscontrollers 8, die Polarisation der Teilstrahlung nunmehr um 90° gedreht ist, wird die verstärkte Teilstrahlung P von Polarisationsstrahlteiler 16 aus der Lichtquelle 1 ausgekoppelt.

Eine Variante des in Figur 12 dargestellten Moduls M ist in Figur 13 illustriert. Zwischen dem zweiten Verstärkermedium 4 und dem Spiegel 10 ist das zweite Filterelement 6 angebracht. Das zweite Filterelement 6 wird somit von der Teilstrahlung P ebenfalls zweifach durchlaufen. Hierdurch ist eine hohe Unterdrückung des spektral breitbandigen Untergrunds der Strahlung R der Ausgangsquelle 2 gewährleistet.

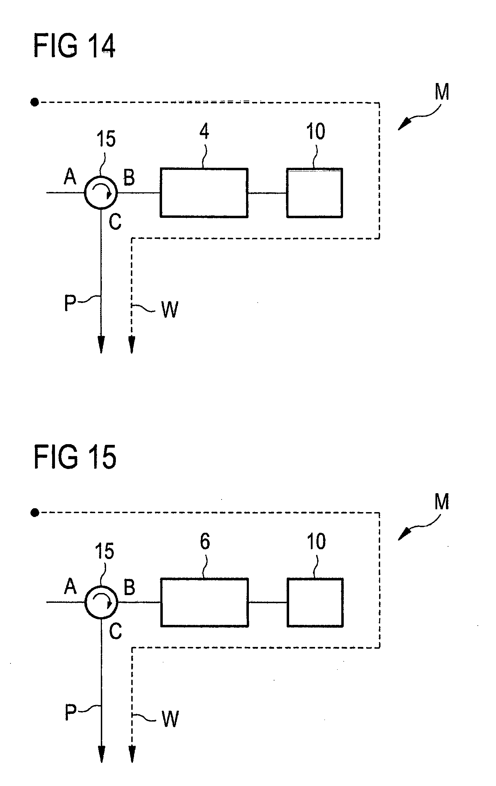

Anstelle des polarisationsselektiven Elements 12 gemäß den Figuren 12 und 13 ist in den Figuren 14 und 15 ein Zirkulator 15 eingesetzt. Das zweite Verstärkermedium 4, siehe Figur 14, kann polarisationsselektiv ausgestaltet sein. Anstelle des zweiten Verstärkermediums 4, das zweifach durchlaufen wird, kann alternativ oder auch zusätzlich ein zweites Filterelement 6 treten, siehe Figur 15. Der Spiegel 10 ist in dieser Anordnung bevorzugt nicht polarisationsselektiv. Das zweite Filterelement 6 wirkt transmissiv-absorptiv, das heißt, nicht transmittiertes Licht wird absorbiert und nicht über Reflexion in Richtung hin zum nicht gezeichneten ersten Verstärkermedium zurück geleitet.

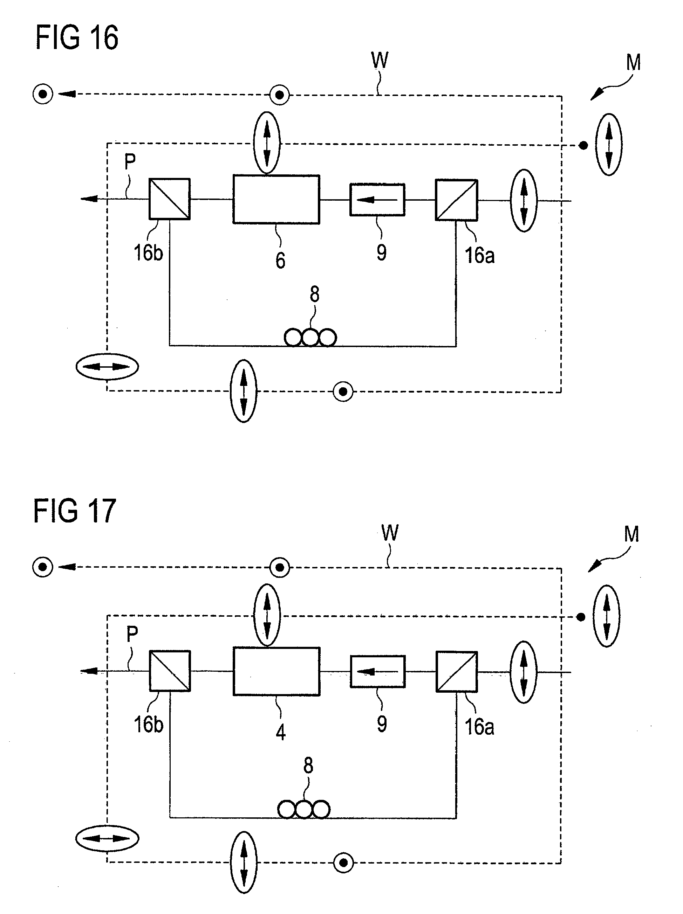

In Figur 16 ist die Verwendung eines transmissiv-reflektiven zweiten Filterelements 6, das zweifach durchlaufen wird, dargestellt. Das in Figur 16 gezeigte Modul M kann beispielsweise anstelle des in Figur 15 dargestellten Moduls M in eine Lichtquelle 1 beispielsweise gemäß Figur 4 oder Figur 10 eingesetzt werden. In das Modul M gemäß Figur 16 wird beispielsweise parallel zur Zeichenebene polarisiertes Licht, symbolisiert durch einen umringten Doppelpfeil, eingekoppelt. Weist die Teilstrahlung P Licht mit zur Zeichenebene senkrechter Polarisation auf, so ist dies durch einen Punkt in einem Kreis symbolisiert. Dieses durchläuft den Polarisationsstrahlteiler 16a und den Isolator 9 und gelangt zum transmissiv-reflektiven zweiten Filterelement 6. Das vom zweiten Filterelement 6 reflektierte, nicht transmittierte Licht wird vom Isolator 9 absorbiert. Über den Polarisationsstrahlteiler 16b wird die Teilstrahlung P reflektiert, durchläuft den Polarisationscontroller 8, der beispielsweise als Faraday-Rotator ausgestaltet ist, wird vom Polarisationsstrahlteiler 16a reflektiert, durchläuft ein weiteres Mal das zweite Filterelement 8 und wird über den Polarisationsstrahlteiler 16b ausgekoppelt.

In Figur 17 ist ein entsprechendes Modul M dargestellt, wobei alternativ, oder auch zusätzlich, zum zweiten Filterelement 6 ein zweites Verstärkermedium 4 eingesetzt ist. Der optische Isolator 9 ist optional und dient zu einer Unterdrückung des spektral breitbandigen Untergrunds, der vom zweiten Verstärkermedium 4 emittiert werden kann.

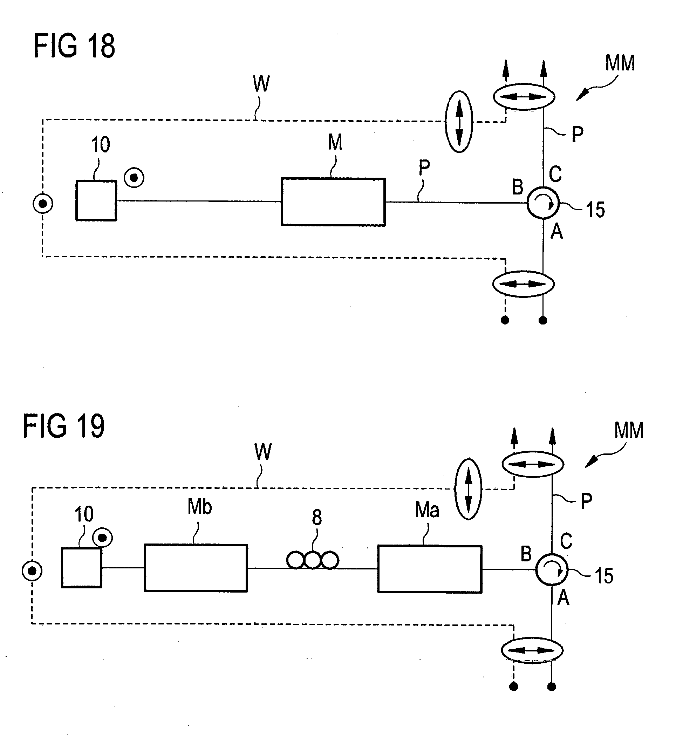

Die vierfache Nutzung eines transmissiv-absorptiven zweiten Filterelements 6 ist in Figur 18 illustriert. Ein zusammengesetztes Modul MM weist ein Modul M auf. Das Modul M kann beispielsweise der in Figur 16 dargestellten Anordnung entsprechen, wobei dann der gemäß Figur 16 vorhandene optische Isolator 9 entfällt. Das zweite Filterelement 6 ist zudem gemäß Figur 18 bevorzugt als transmissiv-absorptives Filterelement gestaltet.

Alternativ oder zusätzlich ist in Figur 18 schematisch die Vierfachnutzung eines polarisationsunabhängigen Verstärkermediums 4 gezeigt. Das Modul M ist dann etwa wie gemäß Figur 17 gestaltet, wobei der in Figur 17 gezeichnete optische Isolator 9 entfällt.

Gemäß Figur 19 weist das zusammengesetzte Modul MM sowohl ein Modul Ma gemäß Figur 16 als auch ein Modul Mb gemäß Figur 17 auf, wobei es sich beim zweiten Verstärkermedium 4 um ein polarisationsunabhängiges Verstärkermedium handelt und beim zweiten Filterelement 6 um einen transmissiv-absorptiven Filter. Die in den Figuren 16 und 17 dargestellten optischen Isolatoren 9 sind jeweils wegzulassen. Zwischen den Modulen kann optional ein Polarisationscontroller angebracht sein.

Die in den Figuren 10 bis 19 dargestellten Ausführungsbeispiele einer Lichtquelle 1 stellen keine abschließende Auflistung dar, vielmehr können mehrere Module M und/oder zusammengesetzte Module MM auch geschachtelt und kombiniert werden, um längere Ketten von beispielsweise Verstärkermedien 3, 4 oder Filterelementen 6 zu erzielen, die mehrfach durchlaufen werden. Je nach den konkreten Anforderungen an die Lichtquelle 1 können auch zusätzliche Verstärkermedien 3, 4 oder Filterelemente 5, 6 in die Lichtquelle 1 integriert werden, ebenso wie zusätzliche Komponenten wie zum Beispiel Schließer, Polarisationscontroller 8 oder Polarisatoren 16.

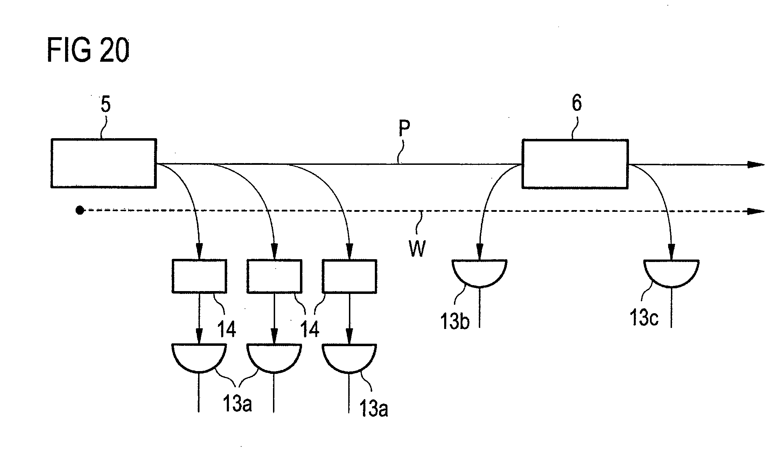

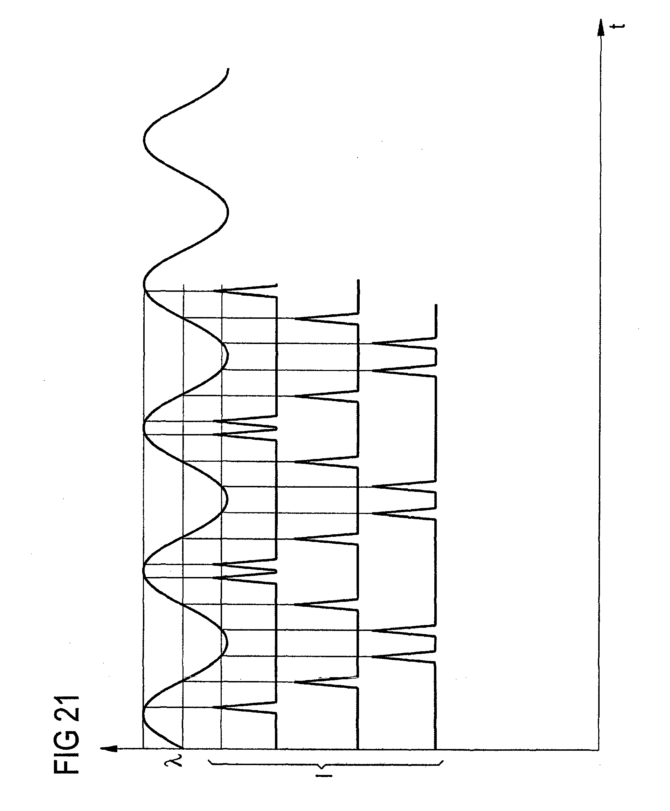

In Figur 20 ist eine Anordnung zur Erzeugung eines Regelsignals zur Ansteuerung der Filterelemente 5, 6 illustriert. Zur Vereinfachung der Darstellung sind beispielsweise die Verstärkermedien 3, 4 nicht gezeichnet. Figur 21 zeigt die Ansteuerung zweier Filterelemente 5, 6, auf eine analoge Weise können auch weitere, zweite Filterelemente 6 angesteuert werden.

Nach dem ersten Filterelement 5 wird ein kleiner Teil der Teilstrahlung P, etwa über einen Faserkoppler, ausgekoppelt. Die ausgekoppelte Teilstrahlung P wird gemäß Figur 20 zu drei separaten Detektoren 13a, die beispielsweise als Fotodioden ausgestaltet sind, geführt. Vor jedem Detektor 13a befindet sich ein wellenlängenselektives Element 14. Die wellenlängenselektiven Elemente 14 sind beispielsweise als Bandpassfilter ausgeführt, die aus einem Faser-Bragg-Gitter in Kombination mit einem Zirkulator oder einer Koppelanordnung bestehen können. Ebenso ist es möglich, dass die wellenlängenselektiven Elemente 14 nur aus einem Faser-Bragg-Gitter in Transmission als Bandstoppfilter gestaltet sind, auch in Kombination mit einem wechselspannungsgekoppelten Detektor 13a. Auch Kantenfilter, beispielsweise auf Farbglasbasis, können Verwendung finden.

Im Falle einer vorbestimmten, zeitlichen periodischen Abstimmung der Wellenlänge der Teilstrahlung P kann über die Detektoren 13a Amplitude, Nullpunktverschiebung und Phase bezüglich des vom ersten Filterelement 5 transmittierten zeitlichen Wellenlängenverlaufs der Teilstrahlung P ermittelt werden. Ein zeitliches Schieben aufgrund Temperaturänderungen oder Schwankungen der Antwortfunktion des ersten Filterelements 5 können ausgeglichen werden.

Zur Synchronisation des zweiten Filterelements 6 auf das erste Filterelement 5 wird ein Teil der vom zweiten Filterelement 6 reflektierten Strahlung auf einen Detektor 13b gelenkt. Alternativ oder zusätzlich wird ein Teil des vom zweiten Filterelement 6 transmittierten Lichts auf einen dem zweiten Filterelement 6 nachgeordneten Detektor 13c geführt. Anders als in Figur 20 dargestellt, können sich vor den Detektoren 13b, 13c optional auch wellenlängenselektive Elemente 14 befinden.

In Figur 21 ist der zeitliche Verlauf der Wellenlänge λ gegenüber der Zeit t aufgetragen, siehe oberste Kurve in Figur 21. Weist die Teilstrahlung P eine bestimmte Wellenlänge auf, so empfängt einer der Detektoren 13a ein Signal mit einer Intensität I, siehe die drei untersten Kurven in Figur 21. Die Wellenlängen, bei denen ein Signal empfangen wird, können über die wellenlängenselektiven Elemente 14 eingestellt werden. Die Signale der Detektoren 13a können über die Steuereinheit zur automatischen Regelung verwendet werden. So kann beispielsweise der Spektralbereich, über den die Teilstrahlung P hinweg abgestimmt wird, und/oder die Intensität der Teilstrahlung P automatisch geregelt werden. Weitere Parameter, die geregelt werden können, sind Treiberspannung, Nullpunktverschiebung oder Phasenverschiebung der Treibersignale.

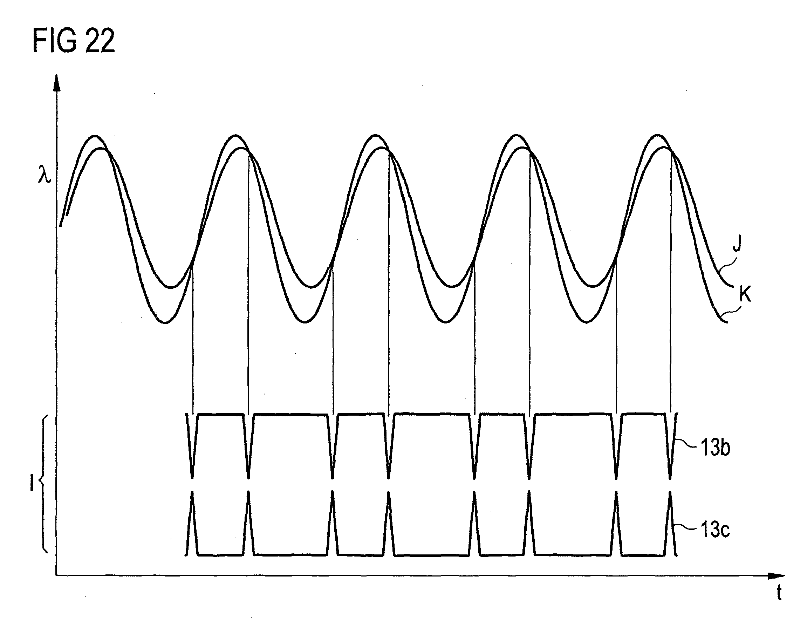

In Figur 22 sind für den Fall, dass erstes Filterelement 5 und zweites Filterelement 6 nicht bezüglich der zeitlichen Verzögerung T aufeinander angepasst sind, in Abhängigkeit von der Zeit t die Signale der Detektoren 13b, 13c gezeigt. In diesem Fall liegt am zweiten Filterelement 6 eine andere, vom ersten Filterelement 5 transmittierte Wellenlänge der Teilstrahlung P an, Kurve K in Figur 22, als die Wellenlänge, die vom zweiten Filterelement 6 zu diesem Zeitpunkt transmittiert wird, siehe Kurve J in Figur 22. Das heißt, die am zweiten Filterelement 6 anliegende Wellenlänge ist gegenüber der vom zweiten Filterelement 6 transmittierten Wellenlänge spektral verschoben. Aufgrund des periodischen Verlaufs der Wellenlängen schneiden sich die beiden Kurven J, K pro Periode nur an zwei Punkten. Diese Schnittpunkte sind durch vertikale, dünne Linien dargestellt.

Schneiden sich beide Kurven J, K, so bedeutet dies, dass die am zweiten Filterelement 6 anliegende Teilstrahlung vom zweiten Filterelement 6 transmittiert wird. Mit anderen Worten wird zu diesem Zeitpunkt weniger oder keine Teilstrahlung vom zweiten Filterelement 6 zurück in Richtung erstes Filterelement 5 reflektiert. Daher bricht am Detektor 13b die Signalintensität ein. Der Detektor 13c zeigt dann ein Signal, wenn vom zweiten Filterelement 6 Strahlung transmittiert wird.

Liegen beide Kurven J, K übereinander, so zeigt der Detektor 13b zu keinem Zeitpunkt ein Signal, während der Detektor 13c immer ein Signal zeigt. Erstes 5 und zweites Filterelement 6 sind also über eine Regelschleife aufeinander automatisch anpassbar, indem beispielsweise das Signal des Detektors 13b minimiert und/oder das Signal des Detektors 13c maximiert wird.

Bei den Ausführungsbeispielen gemäß den Figuren 4 und 10 bis 22 wurden jeweils als Filterelemente 5, 6 Faser-Fabry-Perot-Filter verwendet, die transmissiv-absorptiv oder transmissivreflektiv wirken.

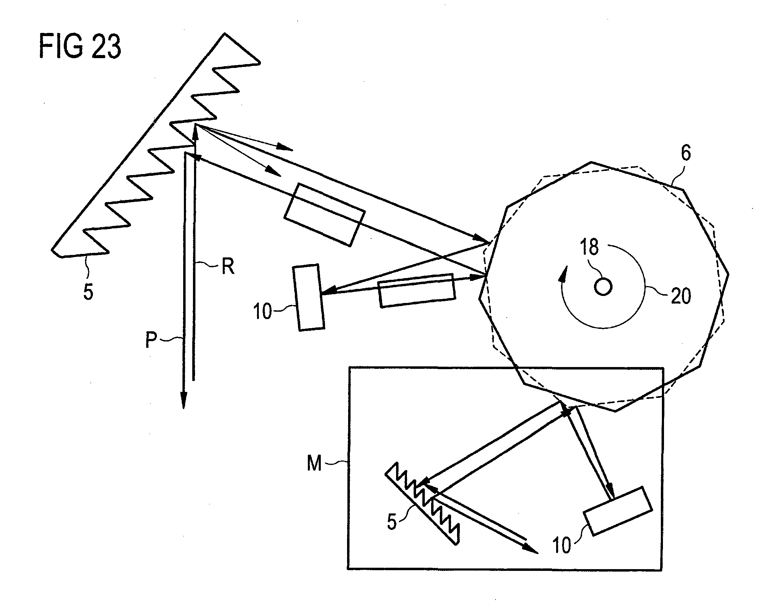

Beim Ausführungsbeispiel gemäß Figur 23 ist eine weitere mögliche Ausgestaltung der Filterelemente 5, 6 illustriert. Die Strahlung R, die beispielsweise von der Ausgangsquelle 2 stammt, die in Figur 23 nicht gezeichnet ist, wird auf ein optisches Beugungsgitter gelenkt, das das erste Filterelement 5 bildet. Vom Beugungsgitter werden verschiedene Wellenlängenkomponenten der Strahlung R in verschiedenen Richtungen reflektiert, siehe dünn gezeichnete Pfeile in Figur 23. Die reflektierte Strahlung R gelangt zu einem um eine Drehachse 18 mit einer Winkelgeschwindigkeit 20 rotierenden Polygonspiegel, der das zweite Filterelement 6 bildet. Die zeitlich variable Stellung des Polygonspiegels ist durch eine Strichlinie symbolisiert. Die Strahlung R trifft hierbei nicht lotrecht auf eine Oberfläche des Polygonspiegels. Vom Polygonspiegel wird die Strahlung R auf den Spiegel 10 reflektiert, der die Strahlung R zurück auf den Polygonspiegel wirft. Vom Polygonspiegel wird die Strahlung R zurück auf das erste Filterelement 5 reflektiert.

Das vom ersten Filterelement 5 in Richtung nicht gezeichneter Ausgangsquelle zurückgeworfene Licht verläuft nur dann parallel zur einfallenden Strahlung R, falls das zweite Filterelement 6 beziehungsweise der Polygonspiegel sich beim zweimaligen Auftreffen der Strahlung R auf den Polygonspiegel jeweils in einer richtigen Position befindet. Ansonsten kann die Strahlung R beziehungsweise die Teilstrahlung P die Filterelemente 5, 6 nicht durchlaufen, ohne herausgefiltert zu werden. Durch das Zusammenspiel von spektral aufspaltendem ersten Filterelement 5 und zeitabhängig unter verschiedenen Winkeln reflektierendem zweiten Filterelement 6 erfolgt also eine Filterung der Strahlung R, so dass zu bestimmten Zeiten nur eine Teilstrahlung P einer bestimmten Wellenlänge durch die Anordnung gelangen kann. Die Abstimmgeschwindigkeit der Filterelemente 5, 6 und der zeitliche Verlauf der Wellenlänge der Teilstrahlung P ist von der Winkelgeschwindigkeit 20 abhängig.

Optional können mehrere Facetten des Polygonspiegels gleichzeitig für mehrere Strahlen zum Filtern verwendet werden, es können also Module M gebildet sein. Da die verschiedenen Facetten des Polygonspiegels zueinander eine feste, zeitlich konstante Phasenbeziehung zueinander aufweisen, ist die Synchronisation der beiden Filterereignisse, jeweils beim Auftreffen auf die verschiedenen Facetten des Polygonspiegels, vereinfacht. Ebenso ist es möglich, dass die Strahlung Verstärkermedien 3, 4 durchläuft, die so platziert sein können, dass nur im richtigen Winkel vom Polygonspiegel reflektierte Teilstrahlung P verstärkt wird.

Da die Winkelgeschwindigkeit des Polygonspiegels sehr präzise einstellbar ist und auch mit hoher Genauigkeit konstant gehalten werden kann, sowie durch die feste Wahl eines Reflexionswinkels am Spiegel 10, ist ein synchrones Abstimmen der Filterereignisse aufeinander beim Auftreffen auf das erste, als Gitter gestaltete Filterelemente 5 beim Vor- und beim Rücklauf der Strahlung R beziehungsweise der Teilstrahlung P auf einfache Weise erreichbar.

Der Polygonspiegel 21 weist beispielsweise einen Durchmesser von 3 bis 4 cm und etwa 20 Facetten auf. Im Gegensatz zu den anderen Ausführungsbeispielen ist die Strahlung R oder die Teilstrahlung P in der Filteranordnung gemäß Figur 23 nicht in einem Lichtleiter geführt. Um beispielsweise ein Auftrennen der in die Anordnung einlaufenden Strahlung R von der auslaufenden Teilstrahlung zu erleichtern, können diese bezüglich der Zeichenebene senkrecht zueinander versetzt sein.

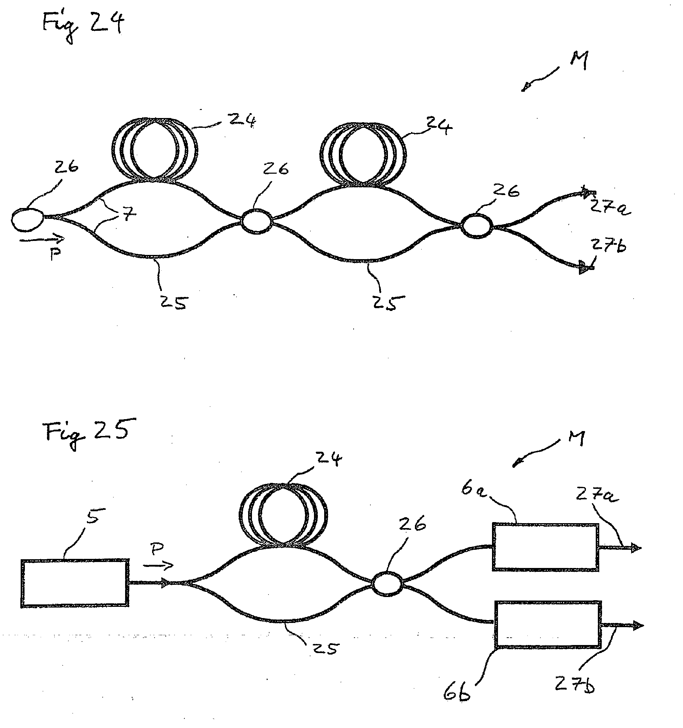

In Figur 24 ist ein weiteres Modul M für ein Ausführungsbeispiel der Lichtquelle schematisch illustriert. Das Modul M weist zwei Verzögerungsstrecken 24 auf, die über Faserkoppler 26 mit Überbrückungsstrecken 25 optisch parallel geschaltet sind. Sowohl die Verzögerungsstrecken 24 als auch die Überbrückungsstrecken 25 sind durch Lichtleiter 7 realisiert. Bevorzugt sind die Verzögerungsstrecken 24 und die Überbrückungsstrecken 25 einem letzten der Filterelemente der Lichtquelle, in Figur 24 nicht dargestellt, nachgeordnet.

Durch die Faserkoppler 26 oder auch durch Strahlteiler wird die Strahlung in die Verzögerungsstrecken 24 sowie in die Überbrückungsstrecken 25 aufgespalten. Die Verzögerungsstrecken 24 gemäß den Figuren 24 und 25 weisen hierbei eine Länge zum Beispiel zwischen einschließlich 100 m und 5 km auf, während hingegen die Überbrückungsstrecken 25 eine deutlich kürzere optische Weglänge aufweisen, beispielsweise weniger als 50 m, insbesondere weniger als 1 m. Die zwei Verzögerungsstrecken 24 können gleiche oder auch voneinander verschiedene optische Längen aufweisen. Insbesondere kann eine der Verzögerungsstrecken 24 eine doppelt so große optische Länge wie die zweite Verzögerungsstrecke haben.

Mit anderen Worten wird die Strahlung P in wenigstens zwei Teile aufgespalten, wobei der Teil der Strahlung P, der durch eine der Verzögerungsstrecken 24 oder durch beide Verzögerungsstrecken 25 läuft, einen Ausgang 27a, 27b des Moduls M oder der Lichtquelle aufgrund der größeren optischen Weglänge später erreicht als der Teil der Strahlung P, der durch die Überbrückungsstrecken 25 läuft. Derselbe Wellenzug wird also bezüglich der Intensität in die Verzögerungsstrecken 24 und die Überbrückungsstrecken 25 aufgeteilt und zeitlich verzögert an den Ausgängen 27a, 27b des Moduls M oder der Lichtquelle emittiert. Hierdurch kann eine Abstimmrate oder eine Wiederholrate von Wellenzügen der Lichtquelle mit einem solchen Modul M erhöht werden.

Das Modul M gemäß Figur 24 weist genau zwei Verzögerungsstrecken 24 auf. Eine Abstimmrate einer Lichtquelle mit einem solchen Modul M gemäß Figur 24 kann also vervierfacht sein. Abweichend von der Darstellung gemäß Figur 24 kann das Modul M auch nur eine Verzögerungsstrecke 24 oder mehr als zwei Verzögerungsstrecken 24 aufweisen. Es ist möglich, dass die zwei Ausgänge 27a, 27b in einen einzigen Ausgang zusammengelegt werden können, anders als in Figur 24 gezeigt. Bevorzugt ist wenigstens einem der Ausgänge 27a, 27b oder einem zusammengelegten Ausgang ein zweites Verstärkermedium nachgeordnet, das in Figur 24 nicht dargestellt ist.

Bei dem Modul M gemäß Figur 25 befindet sich die Verzögerungsstrecke 24, die der Überbrückungsstrecke 24 optisch parallel geschaltet ist, zwischen dem ersten Filterelement 5 und zwei zweiten Filterelementen 6a, 6b. Ein Teil der Strahlung P durchläuft also die Verzögerungsstrecke 24, ein anderer Teil der Strahlung P die Überbrückungsstrecke 25, die im Vergleich zur Verzögerungsstrecke 24 eine kürzere optische Länge aufweist. Durch den Faserkoppler 26, der der Verzögerungsstrecke 24 sowie der Überbrückungsstrecke 25 nachfolgt, können die beiden Teile der Strahlung P zu den zwei zweiten Filterelementen 6a, 6b und zu den Ausgängen 27a, 27b geleitet werden.

Bevorzugt ist zwischen dem ersten Filterelement 5 und mindestens einem der zweiten Filterelemente 6a, 6b ein erstes Verstärkermedium und/oder ein zweites Verstärkermedium, in Figur 25 jeweils nicht dargestellt, angeordnet. Anders als in Figur 25 gezeigt, können die Ausgänge 27a, 27b zusammengelegt sein. Es ist ferner möglich, dass eine Lichtquelle mehrere der Module M aufweist.

Diese Patentanmeldung beansprucht die Priorität der deutschen Patentanmeldung 10 2008 045 634.9.

## Patentansprüche

1. Wellenlängenabstimmbare Lichtquelle (1) mit

   - einer Ausgangsquelle (2), die zur Erzeugung einer elektromagnetischen Strahlung (R) geeignet ist,
   - einem wellenlängenselektiven ersten Filterelement (5), das der Ausgangsquelle (2) nachgeordnet ist,
   - einem ersten Verstärkermedium (3), das dem ersten Filterelement (5) nachgeordnet ist und mindestens zur teilweisen Verstärkung der Strahlung (R) geeignet ist,
   - mindestens einem wellenlängenselektiven zweiten Filterelement (6), das dem ersten Verstärkermedium (3) nachgeordnet ist, wobei das zweite Filterelement (6) zum ersten Filterelement (5) einen optischen Abstand (L) aufweist,

- einer Steuereinheit (11), über die das erste und das mindestens eine zweite Filterelement (5, 6) abstimmbar sind, so dass erstes und zweites Filterelement (5, 6) für eine Teilstrahlung (P) der Strahlung (R) in einer zeitlichen Verzögerung (T) zueinander durchlässig sind, wobei die Verzögerung (T) gleich dem Quotienten aus dem optischen Abstand (L) und der Vakuumlichtgeschwindigkeit (c) ist.

2. Lichtquelle (1) nach Anspruch 1, die mindestens ein zweites Verstärkermedium (4) umfasst.

3. Lichtquelle (1) nach einem der vorhergehenden Ansprüche, bei der die Ausgangsquelle als erstes Verstärkermedium verwendbar ist.

4. Lichtquelle (1) nach einem der vorhergehenden Ansprüche, bei der mindestens ein Filterelement (5, 6) und/oder mindestens ein Verstärkermedium (3, 4) mindestens zweifach von der Teilstrahlung (P) durchlaufbar ist.

5. Lichtquelle (1) nach einem der vorhergehenden Ansprüche, bei der mindestens ein Filterelement (5, 6) und/oder mindestens ein Verstärkermedium (3, 4) mindestens vierfach von der Teilstrahlung (P) durchlaufbar ist.

6. Lichtquelle (1) nach dem vorhergehenden Anspruch, die mindestens zwei polarisationsselektive Elemente (12) umfasst und bei der das Verstärkermedium (3, 4) polarisationsunabhängig ausgestaltet ist.

7. Lichtquelle (1) nach einem der vorhergehenden Ansprüche, bei der die Ausgangsquelle (2), das mindestens eine Verstärkermedium (3, 4) und die Filterelemente (5, 6) über mindestens einen Lichtleiter optisch miteinander verbunden sind.

8. Lichtquelle (1) nach einem der vorhergehenden Ansprüche, bei der das erste Filterelement (5) und/oder das mindestens eine zweite Filterelement (6) ein Faser-Fabry-Perot-Filter ist.

9. Lichtquelle (1) nach einem der vorhergehenden Ansprüche, bei der das erste Filterelement (5) und/oder das mindestens eine zweite Filterelement (6) ein transmissivabsorptiver Filter ist.

10. Lichtquelle (1) nach einem der vorhergehenden Ansprüche, bei der das erste Filterelement (5) und/oder das mindestens eine zweite Filterelement (6) mindestens ein optisches Gitter oder ein optisches Prisma und/oder einen Polygonspiegel umfasst.

11. Lichtquelle (1) nach einem der vorhergehenden Ansprüche, bei der zumindest ein Verstärkermedium (3, 4) als Filterelement (5, 6) verwendbar ist.

12. Lichtquelle (1) nach einem der vorhergehenden Ansprüche, bei der eine spektrale Breite der Strahlung (R) mindestens 20 nm beträgt.

13. Lichtquelle (1) nach einem der vorhergehenden Ansprüche, bei der eine spektrale Breite der Teilstrahlung (P) im Wertebereich zwischen 0,003 nm und 5,0 nm liegt.

14. Lichtquelle (1) nach einem der vorhergehenden Ansprüche, bei der eine optische Kohärenzlänge der Teilstrahlung (P) mindestens 3 mm beträgt.

15. Lichtquelle (1) nach einem der vorhergehenden Ansprüche, bei der eine Zentralwellenlänge der Teilstrahlung (P) während der zeitlichen Verzögerung (T) um mindestens ein Zehntel der spektralen Breite der Teilstrahlung (P) abstimmbar ist.

16. Lichtquelle (1) nach einem der vorhergehenden Ansprüche, bei der eine Zentralwellenlänge der Teilstrahlung (P) während der zeitlichen Verzögerung (T) um mindestens die spektrale Breite der Teilstrahlung (P) abstimmbar ist.

17. Lichtquelle (1) nach einem der vorhergehenden Ansprüche, bei der eine Abstimmgeschwindigkeit mindestens eines der Filterelemente (5, 6) zumindest zeitweise mindestens 0,5 nm/$\mu$s beträgt.

18. Lichtquelle (1) nach einem der vorhergehenden Ansprüche, bei der eine Abstimmgeschwindigkeit mindestens eines der Filterelemente (5, 6) zumindest zeitweise mindestens 3,0 nm/$\mu$s beträgt.

19. Lichtquelle (1) nach einem der vorhergehenden Ansprüche, bei der eine Abstimmfrequenz der Filterelemente (5, 6) mindestens zeitweise zumindest 40 kHz beträgt.

20. Lichtquelle (1) nach einem der vorhergehenden Ansprüche, dessen optische Länge grösser oder gleich 0,5 m ist.

21. Lichtquelle (1) nach einem der vorhergehenden Ansprüche, bei der zumindest ein Verstärkermedium (3, 4) mit mindestens einem optischen Halbleiterverstärker gestaltet ist.

22. Lichtquelle (1) nach einem der Ansprüche 3 bis 21, bei der für eine Sättigungsleistung $P_{sat}$ des ersten und/oder des zweiten Verstärkermediums (3, 4) gilt:

$$P_{sat} > 0{,}1 \; G \; P_A \; (B_P / B_A),$$

wobei G ein Kleinsignalverstärkungsfaktor des ersten und/oder des zweiten Verstärkungsmediums (3, 4), Bp die spektrale Breite der Teilstrahlung (P), $B_A$ die spektrale Breite der Strahlung (R) und $P_A$ eine Gesamtleistung der Strahlung der Ausgangsquelle (2) ist.

23. Lichtquelle (1) nach einem der vorhergehenden Ansprüche, die mindestens einen wellenlängenselektiven Detektor (13) umfasst.

24. Lichtquelle (1) nach dem vorhergehenden Anspruch, bei der die Steuereinheit (11) mit dem mindestens einen Detektor (13) verbunden ist und die Verzögerung (T) durch ein Signal des Detektors (13) einstellbar ist.

25. Lichtquelle (1) nach dem vorhergehenden Anspruch, bei der ein vom zweiten Filterelement (6) reflektierter Anteil der Teilstrahlung (P) vom Detektor (13) detektierbar ist.

26. Lichtquelle (1) nach einem der vorhergehenden Ansprüche, die dazu geeignet ist, Teilstrahlung (P) im nahinfraroten Spektralbereich zu emittieren.

27. Lichtquelle (1) nach einem der vorhergehenden Ansprüche, die dazu geeignet ist, Teilstrahlung (P) einer Wellenlänge von kleiner oder gleich 900 nm zu emittieren.

28. Lichtquelle (1) nach einem der vorhergehenden Ansprüche, bei der die Ausgangsquelle (2) ein Semiconductor Optical Amplifier und bei der das erste Verstärkermedium (3) eine Seltene-Erden-dotierte Glasfaser ist.

29. Lichtquelle (1) nach dem vorhergehenden Anspruch und nach Anspruch 2, bei dem das zweite Verstärkermedium (4) eine Seltene-Erden-dotierte Glasfaser ist.

30. Lichtquelle (1) nach einem der vorhergehenden Ansprüche, die mindestens eine Verzögerungsstrecke (24) umfasst, die einer Überbrückungsstrecke (25) optisch parallel geschaltet ist.

31. Lichtquelle (1) nach dem vorhergehenden Anspruch, bei der die Verzögerungsstrecke (24) dem zweiten Filterelement (6) oder einem letzten der zweiten Filterelemente (6) nachfolgt.

32. Lichtquelle (1) nach Anspruch 30, bei der sich die Verzögerungsstrecke (24) zwischen zwei aufeinander folgenden Filterelementen (5, 6) befindet.

33. Lichtquelle (1) nach einem der vorhergehenden Ansprüche, bei der ein optischer Abstand zwischen der Ausgangsquelle (2) und dem ersten Verstärkermedium (3) und/oder ein optischer Abstand zwischen zwei aufeinander folgenden Verstärkermedien (3, 4) mindestens das 0,2-Fache des Quotienten aus der Vakuumlichtgeschwindigkeit und einer Filterwechselzeit beträgt, wobei die Filterwechselzeit eine Periodendauer der Wellenlängenabstimmung, multipliziert mit einer Filterbandbreite des Filters (5, 6) und dividiert durch die spektrale Breite der von der Lichtquelle emittierten Strahlung (S), ist.

**Claims**

1. A wavelength-tunable light source (1) having

   - an output source (2), which is capable of generating electromagnetic radiation (R),
   - a wavelength-selective first filter element (5), which is situated downstream from the output source (2),
   - a first amplifier medium (3), which is situated downstream from the first filter element (5) and is capable of at least partial amplification of the radiation (R),
   - at least one wavelength-selective second filter element (6), which is situated downstream from the first amplifier medium (3), the second filter element (6) having an optical spacing (L) to the first filter element (5),
   - a control unit (11), via which the first and the at least one second filter elements (5, 6) are tunable, so that first and second filter elements (5, 6) are transparent to a partial radiation (P) of the radiation (R) in a time delay (T) to one another, wherein the delay (T) is equal to the quotient of the optical spacing (L) and the speed of light in vacuum (c).

2. The light source (1) according to Claim 1,
   which comprises at least one second amplifier medium (4).

3. The light source (1) according to one of the preceding claims,
   wherein the output source is used as the first amplifier medium.

4. The light source (1) according to one of the preceding claims,
   wherein the partial radiation (P) can pass through at least one filter element (5, 6) and/or at least one amplifier medium (3, 4) at least twice.

5. The light source (1) according to one of the preceding claims,
   wherein the partial radiation (P) can pass through at least one filter element (5, 6) and/or at least one amplifier medium (3, 4) at least four times.

6. The light source (1) according to the preceding claim,
   which comprises at least two polarization-selective elements (12) and wherein the amplifier medium (3, 4) is implemented as polarization-independent.

7. The light source (1) according to one of the preceding claims,
   wherein the output source (2), the at least one amplifier medium (3, 4), and the filter elements (5, 6) are optically connected to one another via at least one optical fiber.

8. The light source (1) according to one of the preceding claims,
   wherein the first filter element (5) and/or the at least one second filter element (6) are Fibre-Fabry-Perot filters.

9. The light source (1) according to one of the preceding claims,
   wherein the first filter element (5) and/or the at least one second filter element (6) are transmissive-absorptive filters.

10. The light source (1) according to one of the preceding claims,
    wherein the first filter element (5) and/or the at least one second filter element (6) comprise at least one optical grating or an optical prism and/or a polygonal mirror.

11. The light source (1) according to one of the preceding claims,
    wherein at least one amplifier medium (3, 4) is usable as a filter element (5, 6).

12. The light source (1) according to one of the preceding claims,
    wherein a spectral width of the radiation (R) is at least 20 nm.

13. The light source (1) according to one of the preceding claims,
    wherein a spectral width of the partial radiation (P) is in the value range between 0.003 nm and 5.0 nm.

14. The light source (1) according to one of the preceding claims,
    wherein an optical coherence length of the partial radiation (P) is at least 3 mm.

15. The light source (1) according to one of the preceding claims,
wherein a central wavelength of the partial radiation (P) is tunable during the time delay (T) by at least one-tenth of the spectral width of the partial radiation (P).

16. The light source (1) according to one of the preceding claims,
wherein a central wavelength of the partial radiation (P) is tunable during the time delay (T) by at least the spectral width of the partial radiation (P).

17. The light source (1) according to one of the preceding claims,
wherein a tuning speed of at least one of the filter elements (5, 6) is at least temporarily at least 0.5 nm/$\mu$s.

18. The light source (1) according to one of the preceding claims,
wherein a tuning speed of at least one of the filter elements (5, 6) is at least temporarily at least 3.0 nm/$\mu$s.

19. The light source (1) according to one of the preceding claims,
wherein a tuning frequency of the filter elements (5, 6) is at least temporarily at least 40 kHz.

20. The light source (1) according to one of the preceding claims, whose optical length is greater than or equal to 0.5 m.

21. The light source (1) according to one of the preceding claims,
wherein at least one amplifier medium (3, 4) is implemented using at least one optical semiconductor amplifier.

22. The light source (1) according to one of Claims 3 through 21,
wherein the following relationship applies for a saturation power $P_{sat}$ of the first and/or the second amplifier medium (3, 4):

$$P_{sat} > 0.1\ G\ P_A\ (B_P/B_A),$$

G being a small amplification factor of the first and/or the second amplifier media (3, 4), $B_P$ being the spectral width of the partial radiation (P), and $B_A$ being the spectral width of the radiation (R), and $P_A$ being a total power of the radiation of the output source (2).

23. The light source (1) according to one of the preceding claims,
which comprises at least one wavelength-selective detector (13).

24. The light source (1) according to the preceding claim,
wherein the control unit (11) is connected to the at least one detector (13) and the delay (T) is settable by a signal of the detector (13).

25. The light source (1) according to the preceding claim,
wherein a component of the partial radiation (P) reflected by the second filter element (6) is detectable by the detector (13).

26. The light source (1) according to one of the preceding claims,
which is capable of emitting partial radiation (P) in the near-infrared spectral range.

27. The light source (1) according to one of the preceding claims,
which is capable of emitting partial radiation (P) of a wavelength of less than or equal to 900 nm.

28. The light source (1) according to one of the preceding claims,
wherein the output source (2) is a semiconductor optical amplifier, and the first amplifier medium (3) is a rare-earth-doped glass fiber.

29. The light source (1) according to the preceding claim and according to Claim 2,
wherein the second amplifier medium (4) is a rare-earth-doped glass fiber.

**30.** The light source (1) according to one of the preceding claims,
which comprises at least one delay route (24), which is connected optically in parallel to a bypass route (25).

**31.** The light source (1) according to the preceding claim,
wherein the delay route (24) follows the second filter element (6) or a last of the second filter elements (6).

**32.** The light source (1) according to Claim 30,
wherein the delay route (24) is located between two sequential filter elements (5, 6).

**33.** The light source (1) according to one of the preceding claims,
wherein an optical spacing between the output source (2) and the first amplifier medium (3) and/or an optical spacing between two sequential amplifier media (3, 4) is at least 0.2 times the quotient of the speed of light in vacuum and a filter changing time,
the filter changing time being a period duration of the wavelength tuning, multiplied by a filter bandwidth of the filter (5, 6), and divided by the spectral width of the radiation (S) emitted by the light source.

**Revendications**

**1.** Source lumineuse (1) adaptable en longueur d'onde, comprenant

- une source de sortie (2), qui se prête à générer un rayonnement électromagnétique (R),
- un premier élément filtrant (5) sélectif de longueur d'onde, monté en aval de la source de sortie (2),
- un premier milieu amplificateur (3), monté en aval du premier élément filtrant (5) et qui se prête au moins à l'amplification partielle du rayonnement (R),
- au moins un second élément filtrant (6) sélectif de longueur d'onde, monté en aval du premier milieu amplificateur (3), le second élément filtrant (6) présentant une distance optique (L) par rapport au premier élément filtrant (5),
- une unité de commande (11), par l'intermédiaire de laquelle le premier et le au moins un second élément filtrant (5, 6) sont accordables, de sorte que le premier et le second élément filtrant (5, 6) sont mutuellement perméables pour un rayonnement partiel (P) du rayonnement (R) en un décalage temporel(T), le décalage temporel(T) étant égal au quotient de la distance optique (L) et de la vitesse de la lumière dans le vide (c).

**2.** Source lumineuse (1) selon la revendication 1, qui comporte au moins un second milieu amplificateur (4).

**3.** Source lumineuse (1) selon l'une des revendications précédentes, dans laquelle la source de sortie est utilisable en tant que premier milieu amplificateur.

**4.** Source lumineuse (1) selon l'une des revendications précédentes, dans laquelle au moins un élément filtrant (5, 6) et/ou au moins un milieu amplificateur (3, 4) peuvent être traversés au moins deux fois par le rayonnement partiel (P).

**5.** Source lumineuse (1) selon l'une des revendications précédentes, dans laquelle au moins un élément filtrant (5, 6) et/ou au moins un milieu amplificateur (3, 4) peuvent être traversés au moins quatre fois par le rayonnement partiel (P).

**6.** Source lumineuse (1) selon la revendication précédente, qui comporte au moins deux éléments (12) sélectifs de polarisation et dans laquelle le milieu amplificateur (3, 4) a une configuration indépendante de la polarisation.

**7.** Source lumineuse (1) selon l'une des revendications précédentes, dans laquelle la source de sortie (2), le au moins un milieu amplificateur (3, 4) et les éléments filtrants (5, 6) sont reliés optiquement entre eux par l'intermédiaire d'au moins un guide de lumière.

**8.** Source lumineuse (1) selon l'une des revendications précédentes, dans laquelle le premier élément filtrant (5) et/ou le au moins un second élément filtrant (6) est un filtre à fibres de Fabry-Perot.

**9.** Source lumineuse (1) selon l'une des revendications précédentes, dans laquelle le premier élément filtrant (5) et/ou le au moins un second élément filtrant (6) est un filtre à absorption transmission.

**10.** Source lumineuse (1) selon l'une des revendications précédentes, dans laquelle le premier élément filtrant (5) et/ou le au moins un second élément filtrant (6) comporte au moins une grille optique ou un prisme optique et/ou un miroir polygonal.

11. Source lumineuse (1) selon l'une des revendications précédentes, dans laquelle au moins un milieu amplificateur (3, 4) est utilisable en tant qu'élément filtrant (5, 6).

12. Source lumineuse (1) selon l'une des revendications précédentes, dans laquelle une largeur spectrale du rayonnement (R) est au moins égale à 20 nm.

13. Source lumineuse (1) selon l'une des revendications précédentes, dans laquelle une largeur spectrale du rayonnement partiel (P) se situe dans une plage de valeurs comprises entre 0,003 nm et 5,0 nm.

14. Source lumineuse (1) selon l'une des revendications précédentes, dans laquelle une longueur de cohérence optique du rayonnement partiel (P) est au moins égale à 3 mm.

15. Source lumineuse (1) selon l'une des revendications précédentes, dans laquelle une longueur d'onde centrale du rayonnement partiel (P) est accordable, pendant le décalage temporel (T), d'au moins un dixième de la largeur spectrale du rayonnement partiel (P).

16. Source lumineuse (1) selon l'une des revendications précédentes, dans laquelle une longueur d'onde centrale du rayonnement partiel (P) est accordable, pendant le décalage temporel (T), d'au moins la largeur spectrale du rayonnement partiel (P).

17. Source lumineuse (1) selon l'une des revendications précédentes, dans laquelle une rapidité d'accord d'au moins l'un des éléments filtrants (5, 6) est égale, au moins par intermittence, à au moins 0,5 nm/$\mu$s.

18. Source lumineuse (1) selon l'une des revendications précédentes, dans laquelle une rapidité d'accord d'au moins l'un des éléments filtrants (5, 6) est égale, au moins par intermittence, à au moins 3,0 nm/$\mu$s.

19. Source lumineuse (1) selon l'une des revendications précédentes, dans laquelle une fréquence d'accord des éléments filtrants (5, 6) est égale, au moins par intermittence, à au moins 40 kHz.

20. Source lumineuse (1) selon l'une des revendications précédentes, dont la longueur optique est supérieure ou égale à 0,5 m.

21. Source lumineuse (1) selon l'une des revendications précédentes, dans laquelle au moins un milieu amplificateur (3, 4) est configuré avec au moins un amplificateur optique à semi-conducteur.

22. Source lumineuse (1) selon l'une des revendications 3 à 21, dans laquelle s'applique la relation pour une puissance de saturation $P_{sat}$ du premier et/ou du second milieu amplificateur (3, 4) :

$$P_{sat} > 0,1 \ G \ P_A \ (B_p/B_A),$$

G étant un facteur d'amplification de petits signaux du premier et/ou du second milieu amplificateur (3, 4), $B_p$ la largeur spectrale du rayonnement partiel (P), $B_A$ la largeur spectrale du rayonnement (R) et $P_A$ une puissance totale du rayonnement de la source de sortie (2).

23. Source lumineuse (1) selon l'une des revendications précédentes, qui comporte au moins un détecteur (13) sélectif de longueur d'onde.

24. Source lumineuse (1) selon la revendication précédente, dans laquelle l'unité de commande (11) est reliée à le au moins un détecteur (13) et le décalage temporel (T) est réglable par un signal du détecteur (13).

25. Source lumineuse (1) selon la revendication précédente, dans laquelle une partie du rayonnement partiel (P), réfléchie par le second élément filtrant (6), est détectable par le détecteur (13).

**26.** Source lumineuse (1) selon l'une des revendications précédentes, qui se prête à émettre un rayonnement partiel (P) dans le domaine spectral de l'infrarouge proche.

**27.** Source lumineuse (1) selon l'une des revendications précédentes, qui se prête à émettre un rayonnement partiel (P) d'une longueur d'onde inférieure ou égale à 900 nm.

**28.** Source lumineuse (1) selon l'une des revendications précédentes, dans laquelle la source de sortie (2) est un amplificateur optique à semi-conducteur et dans laquelle le premier milieu amplificateur (3) est une fibre de verre dopée aux terres rares.

**29.** Source lumineuse (1) selon la revendication précédente et selon la revendication 2, dans laquelle le second milieu amplificateur (4) est une fibre de verre dopée aux terres rares.

**30.** Source lumineuse (1) selon l'une des revendications précédentes, qui comporte au moins un circuit de décalage temporel (24), monté en parallèle optique d'un circuit de pontage (25).

**31.** Source lumineuse (1) selon la revendication précédente, dans laquelle le circuit de décalage temporel (24) est placé en aval du second élément filtrant (6) ou d'un dernier des seconds éléments filtrants (6).

**32.** Source lumineuse (1) selon la revendication 30, dans laquelle le circuit de temporisation (24) se situe entre deux éléments filtrants (5, 6) successifs.

**33.** Source lumineuse (6) selon l'une des revendications précédentes, dans laquelle une distance optique entre la source de sortie (2) et le premier milieu amplificateur (3) et/ou une distance optique entre deux milieux amplificateurs (3, 4) successifs est au moins égale à 0,2 fois le quotient de la vitesse de la lumière dans le vide et d'une durée de changement de filtre, la durée de changement de filtre étant une durée de période de l'accord de longueur d'onde, multipliée par une largeur de bande passante du filtre (5, 6) et divisée par la largeur spectrale du rayonnement (S) émis par la source lumineuse.

FIG 1

FIG 2

FIG 3

FIG 4

FIG 5

EP 2 364 106 B1

FIG 6

## FIG 7

EP 2 364 106 B1

FIG 8

D

F

G

E

Tp

2 μs

t

0

FIG 9

A)

B)

EP 2 364 106 B1

FIG 10

FIG 11

FIG 12

P

W

FIG 13

P

W

FIG 14

FIG 15

FIG 16

FIG 17

EP 2 364 106 B1

FIG 18

FIG 19

36

FIG 20

37

EP 2 364 106 B1

FIG 21

FIG 22

FIG 23

EP 2 364 106 B1

Fig 24

M

24
24
26
26
26
27a
7
27b
P
25
25

Fig 25

M
24
6a
27a
5
26
P
6b
25
27b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080165366 A1
- DE 102008045634